# EUROPEAN PATENT APPLICATION

(11) **EP 3 020 703 A1**
(43) Date of publication of application: **18.05.2016**
(21) Application number: 14192710.3
(22) Date of filing: 11.11.2014
(51) Int. Cl.: C07C 329/10, C07D 333/24, C07D 207/34, C07D 209/48

(54) **Fluoromalonyl Halfthioesters**

(71) Applicant: ETH Zurich, 8092 Zurich (CH)
(72) Inventor: Wennemers, Helma, 8001 Zürich (CH); Saadi, Jakub, 8046 Zürich (CH)

(57) **Abstract**

The present invention relates to Fluoromalonyl Halfthioesters (FMAHTs) of formula (I), 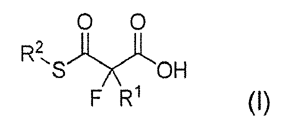 wherein R¹ represents hydrogen, halogen, an optionally substituted alkyl group, an optionally substituted cycloalkyl group, an optionally substituted aryl group; and R² represents an optionally substituted aryl group, an optionally substituted cycloalkyl group, an optionally substituted heteroaryl group, an optionally substituted heterocyclyl group; an optionally substituted alkyl group. The invention further relates to their synthesis and to their use, particularly as masked fluoroacetates in the preparation of pharmaceutical active ingredients.

## Description

The present invention relates to Fluoromalonyl Halfthioesters (FMAHTs), to their synthesis and to their use, particularly as masked fluoroacetates in the preparation of pharmaceutical active ingredients.

It is generally accepted that medicinal chemistry frequently improves the efficacy of pharmaceuticals by introduction of fluorine. Due to the unique influence it has on the properties of organic molecules in biological settings, 25% of all therapeutics contain fluorinated fragments. The stereoselective installation of fluorine is therefore very important but is also highly challenging. Specifically, no efficient methodologies are available to selectively introduce fluorine into important classes of drugs, such as polyketides, cytostatics, anticholesteremic, antifungals, antiparasitics, animal growth promoters.

In consequence, there is a need for compounds suitable to introduce fluorine in pharmaceutically active compounds and for corresponding manufacturing methods.

Chang et al, (Science, 2013, 341, 1089-1094) describe an enzymatic synthesis of fluoromalonic-Coenzyme-A. However the authors use an enzymatic preparation method that is tedious and limited to the synthesis of one specific Coenzyme-A derived FMAHT. The stability of this compound at room temperature is granted only for 24h. The document further discloses one example of ß-hydroxy-α-fluorothioester which is prepared from fluoromalonic- Coenzyme-A in an enzymatic way.

Thus, an object of the present invention is to mitigate at least some of these drawbacks of the state of the art. In particular, it is an aim of the present invention to provide efficient and easily-scalable preparation of novel fluoromalonic acid halfthioesters (FMAHTs), which act as surrogates of fluoroacetate and fluoroacetaldehyde enolates in reactions with electrophiles.

These objectives are achieved by the compound as defined in claim 1 and the methods as defined in claim 9. Further aspects of the invention are disclosed in the specification and independent claims, preferred embodiments are disclosed in the specification and the dependent claims.

The invention may be more fully appreciated by reference to the following description, including the following **glossary of terms** and the concluding examples. For the sake of brevity, the disclosures of the publications cited in this specification are herein incorporated by reference. As used herein, the terms "including", "containing" and "comprising" are used herein in their open, non-limiting sense.

Any formula given herein is intended to represent compounds having structures depicted by the structural formula as well as certain variations or forms. In particular, compounds of any formula given herein may have stereogenic centers and therefore exist in different enantiomeric and diastereoisomeric forms. If at least one stereogenic carbon atom is present in a compound of the formula I, such a compound may exist in optically active form or in the form of a mixture of optical isomers, e. g. in the form of a racemic mixture. All optical isomers and their mixtures, including the racemic mixtures, are part of the present invention. Thus, any formula given herein is intended to represent a racemate, one or more enantiomeric forms, one or more diastereomeric forms, one or more atropisomeric forms, and mixtures thereof. Furthermore, certain structures may exist as geometric isomers (i.e. cis and trans isomers), as tautomers, or as atropisomers. Additionally, any formula given herein is intended to represent hydrates, solvates, and polymorphs of such compounds, and mixtures thereof.

Any formula given herein is also intended to represent unlabeled forms as well as isotopically labeled forms of the compounds. Isotopically labeled compounds have structures depicted by the formulas given herein except that one or more atoms are replaced by an atom having a selected atomic mass or mass number. Examples of isotopes that can be incorporated into compounds of the invention include isotopes of hydrogen, carbon, nitrogen, oxygen, phosphorous, fluorine, and chlorine, such as ²H, ³H, ¹¹C, ¹³C, ¹⁴C, ¹⁵N, ¹⁸F ³¹P, ³²P, ³⁵S, ³⁶Cl, ¹²⁵J respectively. Various isotopically labeled compounds of the present invention, for example those into which radioactive isotopes such as ³H, ¹³C, and ¹⁴C are incorporated. Such isotopically labeled compounds are useful in metabolic studies (preferably with ¹⁴C), reaction kinetic studies (with, for example ²H or ³H), detection or imaging techniques such as positron emission tomography (PET) or single-photon emission computed tomography (SPECT) including drug or substrate tissue distribution assays, or in radioactive treatment of patients. In particular, an ¹⁸F or labeled compound may be particularly preferred for PET or SPECT studies. Further, substitution with heavier isotopes such as deuterium (i.e., ²H) may afford certain therapeutic advantages resulting from greater metabolic stability, for example increased in vivo half-life or reduced dosage requirements. Isotopically labeled compounds of this invention and prodrugs thereof can generally be prepared by carrying out the procedures disclosed in the schemes or in the examples and preparations described below by substituting a readily available isotopically labeled reagent for a non-isotopically labeled reagent.

When referring to any formula given herein, the selection of a particular moiety from a list of possible species for a specified variable is not intended to define the moiety for the variable appearing elsewhere. In other words, where a variable appears more than once, the choice of the species from a specified list is independent of the choice of the species for the same variable elsewhere in the formula.

The following general definitions shall apply in this specification, unless otherwise specified:

The term "halogen" (or halo) denotes fluorine, bromine, chlorine or iodine, preferably fluorine, chlorine.

The term "alkyl" refers to a straight-chain or branched-chain alkyl group, preferably represents a straight-chain or branched-chain C₁₋₁₂alkyl, particularly preferably represents a straight-chain or branched-chain C₁₋₆alkyl; for example, methyl, ethyl, nor iso-propyl, n-, iso-, sec- or tert-butyl, n-pentyl, n-hexyl, n-heptyl, n-octyl, n-nonyl, n-decyl, n-undecyl, n-dodecyl, with particular preference given to methyl, ethyl, n-propyl, iso-propyl, n-butyl and tert-butyl. Alkyl may be unsubstituted or substituted. Exemplary substituents include, but are not limited to hydroxyl, alkoxy, halogen, alkenyl, alkynyl, aryl, and amino. Examples of substituted alkyl include benzyl, allyl, and propargyl. Further, cyclocalkyl may be a substituent to alkyl. An example of such a case is the moiety (alkyl)-cyclopropyl or alkandiyl-cycloproyl, e.g. -CH₂-cyclopropyl.

The term "alkenyl" refers to a straight-chain or branched-chain alkenyl group preferably C₂₋₆ alkenyl, for example, vinyl, allyl, 1-propenyl, isopropenyl, 2-butenyl, 2-pentenyl, 2-hexenyl. In one embodiment, alkenyl preferably represents unsubstituted C₂₋₄ alkenyl. In a further embodiment, alkenyl is substituted by one or more substituents selected from the group consisting of halogen, nitro, cyano, alkyl, alkandiyl, alkenediyl, alkoxy, alkoxyalkyl, alkoxycarbonyl, alkoxycarbonylalkyl, halogenalkyl, aryl, aryloxy, and arylalkyl.

The term "alkynyl" refers to a straight-chain or branched-chain alkyne group, preferably C₂₋₆ alkynyl, for example, propargyl. Preferably, akynyl is unsubstituted. Each alkyl part of "alkoxy", "alkoxyalkyl", "alkoxycarbonyl", "alkoxycarbonylalkyl" and "halogenalkyl" shall have the same meaning as described in the above-mentioned definition of "alkyl".

The term "alkandiyl" refers to a straight-chain or branched-chain alkandiyl group bound by two different Carbon atoms to the moiety, it preferably represents a straight-chain or branched-chain C₁₋₁₂ alkandiyl, particularly preferably represents a straight-chain or branched-chain C₁₋₆ alkandiyl; for example, methandiyl (-CH₂-), 1,2-ethanediyl (-CH₂-CH₂-), 1,1-ethanediyl ((-CH(CH₃)-), 1,1-, 1,2-, 1,3-propanediyl and 1,1-, 1,2-, 1,3-, 1,4-butanediyl, with particular preference given to methandiyl, 1,1-ethanediyl, 1,2-ethanediyl, 1,3-propanediyl, 1,4-butanediyl.

The term "alkendiyl" refers to a straight-chain or branched-chain alkendiyl group bound by two different Carbon atoms to the molecule, it preferably represents a straight-chain or branched-chain C₂₋₆ alkendiyl; for example, -CH=CH-, -CH=C(CH₃)-, -CH=CH-CH₂-, -C(CH₃)=CH-CH₂-, -CH=C(CH₃)-CH₂-, -CH=CH-C(CH₃)H-, -CH=CH-CH=CH-,-C(CH₃)=CH-CH=CH-, -CH=C(CH₃)-CH=CH-, with particular preference given to-CH=CH-CH₂-, -CH=CH-CH=CH-. Alkendiyl may be substituted or unsubstituted

The term "cycloalkyl" refers to a saturated or partially saturated, monocyclic, fused polycyclic, or spiro polycyclic, carbocycle having from 3 to 12 ring atoms per carbocycle. Illustrative examples of cycloalkyl groups include the following moieties: cyclopropyl, cyclobutyl, cyclopentyl and cylclohexyl.

The term "aryl" is known in the field. Aryl is preferably naphthyl or phenyl, in particular phenyl. Aryl may be unsubstituted or substituted, such as by one or more substituents selected from the group consisting of, halogen, nitro, cyano, alkyl, alkandiyl, alkenediyl, alkoxy, alkoxyalkyl, alkoxycarbonyl, alkoxycarbonylalkyl, halogenalkyl, aryl, aryloxy, arylalkyl.

The term "heterocyclyl" refers to a saturated or partly saturated ring system containing at least one hetero atom. Preferably, heterocyclyl groups consist of 3 to 10 ring atoms of which 1-3 ring atoms are hetero atoms. Heterocycles may be present as a single ring system or as bicyclic or tricyclic ring systems; preferably as single ring system or as benz-annelated ring system. Bicyclic or tricyclic ring systems may be formed by annelation of two or more rings, by a bridging atom, e.g. Oxygen, sulfur, nitrogen or by a bridging group, e.g. alkandediyl or alkenediyl. A Heterocycle may be substituted by one or more substituents selected from the group consisting of Oxo (=O), halogen, nitro, cyano, alkyl, alkandiyl, alkenediyl, alkoxy, alkoxyalkyl, alkoxycarbonyl, alkoxycarbonylalkyl, halogenalkyl, aryl, aryloxy, arylalkyl.

The term "heteroaryl" refers to an aromatic ring system containing at least one hetero atom. Preferably, heteroaryl groups consist of 3 to 10 ring atoms of which 1-3 ring atoms are hetero atoms. Heteroary groups may be present as a single ring system or as bicyclic or tricyclic ring systems; preferably as single ring system or as benz-annelated ring system. Bicyclic or tricyclic ring systems may be formed by annelation of two or more rings. A Heterocycle may be substituted by one or more substituents selected from the group consisting of Oxo (=O), halogen, nitro, cyano, alkyl, alkandiyl, alkenediyl, alkoxy, alkoxyalkyl, alkoxycarbonyl, alkoxycarbonylalkyl, halogenalkyl, aryl, aryloxy, arylalkyl. Examples of heterocyclyl and heteroaryl groups include: pyrrole, pyrroline, pyrrolidine, pyrazole, pyrazoline, pyrazolidine, imidazole, imidazoline, imidazolidine, triazole, triazoline, triazolidine, tetrazole, furane, dihydrofurane, tetrahydrofurane, furazane (oxadiazole), dioxolane, thiophene, dihydrothiophene, tetrahydrothiophene, oxazole, oxazoline, oxazolidine, isoxazole, isoxazoline, isoxazolidine, thiazole, thiazoline, thiazlolidine, isothiazole, istothiazoline, isothiazolidine, thiadiazole, thiadiazoline, thiadiazolidine, pyridine, piperidine, pyridazine, pyrazine, piperazine, triazine, pyrane, tetrahydropyrane, thiopyrane, tetrahydrothiopyrane, oxazine, thiazine, dioxine, morpholine, purine, pterine, and the corresponding benz-annelated heterocycles, e.g. indole, isoindole, cumarine, cumaronecinoline, isochinoline, cinnoline.

The term "arylalkyl" refers to an aryl group bound to the molecule via an alkyl group, such as a methyl or ethyl group, preferably phenethyl or benzyl, in particular benzyl. Similarly, cycloalkylalkyl and heterocyclyl represents a cycloalkyl group bound to the molecule via an alkyl group or a heterocyclyl group bound to the molecule via an alkyl group.

Carbon containing groups, moieties or molecules contain 1 to 8, preferably 1 to 6, more preferably 1 to 4, most preferably 1 or 2, carbon atoms. Any non-cyclic carbon containing group or moiety with more than 1 carbon atom is straight-chain or branched.

Hetero atoms are atoms other than Carbon and Hydrogen, preferably nitrogen (N), oxygen (O) or sulfur (S).

Halogen-substituted groups and moieties, such as alkyl substituted by halogen (halogenalkyl) can be mono-, poly- or per-halogenated.

In preferred embodiments, which are preferred independently, collectively or in any combination or sub-combination, the invention relates to a compound of the formula I wherein the substituents are as defined herein.

In more general terms, in a **first aspect,** the invention relates to a compound of formula (I), 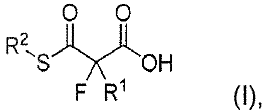 wherein
- R¹: represents hydrogen, halogen, an optionally substituted alkyl group, an optionally substituted cycloalkyl group, an optionally substituted aryl group; and
- R²: represents an optionally substituted aryl group, an optionally substituted cycloalkyl group, an optionally substituted heteroaryl group, an optionally substituted heterocyclyl group; an optionally substituted alkyl group.

The compounds of formula (I) belong to the group of Fluoromalonyl Halfthioesters (FMAHTs). The compounds of formula (I) being surrogates of unsubstituted and substituted fluorothioacetates are particularly useful, as they allow the introduction of fluorine atoms into important classes of pharmaceutically active ingredients, e.g. fluorinated polyketide derivatives and fluorinated carbohydrate derivatives. The compounds of formula (I) are available through scalable preparation methods, as described below and are storable and bench-stable.

This aspect of the invention shall be explained in further detail below:
- R¹: preferably represents hydrogen, halogen, a (C₁-C₈)alkyl group, a (C₃-C₈)cycloalkyl or an aryl group;
wherein said (C₁₋₈)alkyl group is unsubstituted or mono-, di-, tri or tetra-substituted, the optional substituent(s) on the said (C₁₋₈)alkyl moiety being independently selected from the group consisting of halogen, cyano, oxo, nitro, amino, (C₂₋₄)alkenyl, (C₂₋₄)alkynyl, (C₁₋₈)alkoxy, (C₁₋₈)alkoxy(C₁₋₈)alkoxy, (C₁₋₈)alkylthio, (C₁₋₈)alkylsulfinyl, (C₁₋₈) alkylsulfonyl, (C₁₋₈)alkylcarbonyloxy, (C₁₋₈)-alkoxycarbonyl and (C₁₋₈)alkoxy carbonyloxy, (C₃₋₈)cycloalkyl, (C₃₋₈)cycloalkyl(C₁₋₈)alkyl, (C₃₋₈)cycloalkoxy, (C₃₋₈)cycloalkoxy(C₁₋₈)alkyl, (C₃₋₈)cycloalky)(C₁₋₈)alkoxy, (C₃₋₈)cycloalkoxy(C₁₋₈)alkoxy, aryl, aryl(C₁₋₈)alkyl, aryloxy, aryloxy(C₁₋₈)alkyl, aryl(C_{1**-**8})alkoxy, aryloxy(C₁₋₈)alkoxy, carboxy, carbamyl, hydroxy, (C₁₋₈)alkoxy, (C₁₋₈)alkoxy(C₁₋₈)alkoxy, (C₁₋₈)alkoxy substituted by halogen, (C₁₋₈)alkoxy(C₁₋₈)alkyl, (C₁₋₈)alkylthio, (C₁₋₈)alkylthio(C₁₋₈)alkyl, (C₁₋₈)alkylsulfinyl, (C₁₋₈)alkylsulfinyl(C₁₋₈)alkyl, (C₁₋₈)alkylsulfonyl, (C₁₋₈)alkylsulfonyl(C₁₋₈)alkyl, (C₁₋₈)alkylamino, di(C₁₋₈)alkylamino with two identical or different (C₁₋₈)alkyl moieties, amino(C₁₋₈)alkyl, (C₁₋₈)alkylamino(C₁₋₈)alkyl, di(C₁₋₈)alkylamino(C₁₋₈)alkyl with two identical or different (C₁₋₈)alkyl moieties in the di(C₁₋₈)alkylamino moiety, amino(C₁₋₈)alkoxy, (C₁₋₈)alkylamino(C₁₋₈)alkoxy, di(C₁₋₈)alkylamino(C₁₋₈)alkoxy with two identical or different (C₁₋₈)alkyl moieties, formyl, (C₁₋₈)alkylcarbonyl, formyloxy, (C₁₋₈)alkylcarbonyloxy, formyl(C₁₋₈)alkyl, (C₁₋₈)alkylcarbonyl(C₁₋₈)alkyl, formyl(C₁₋₈)alkoxy, (C₁₋₈)alkylcarbonyl(C₁₋₈)alkoxy, (C₁₋₈)alkoxycarbonyl, (C₁₋₈)alkoxycarbonyloxy, (C₁₋₈)alkoxycarbonyl(C₁₋₈)alkyl, (C₁₋₈)alkoxycarbonyl(C₁₋₈)alkoxy and
wherein said aryl group, (C₃-C₈)cycloalkyl group is unsubstituted, mono-substituted, di-substituted or tetra- substituted, the optional substituent(s) being independently selected from the group consisting of halogen, cyano, nitro, carboxy, carbamyl, hydroxy, (C₁₋₈)alkyl, (C₁₋₈)alkyl substituted by halogen, (C₃₋₈)cycloalkyl, (C₃₋₈)cycloalkyl(C₁₋₈)alkyl, (C₃₋₈)cycloalkoxy, (C₃₋₈)cycloalkoxy(C₁₋₈)alkyl, (C₃₋₈)cycloalkyl(C₁₋₈)alkoxy, (C₃₋₈)cycloalkoxy(C₁₋₈)alkoxy, aryl, aryl(C₁₋₈)alkyl, aryloxy, aryloxy(C₁₋₈)alkyl, aryl(C₁₋₈)alkoxy, aryloxy(C₁₋₈)alkoxy, (C₁₋₈)alkoxy, (C₁₋₈)alkoxy(C₁₋₈)alkoxy, (C₁₋₈)alkoxy substituted by halogen, (C₁₋₈)alkoxy(C₁₋₈)alkyl, (C₁₋₈)alkylthio, (C₁₋₈)alkylthio(C₁₋₈)alkyl, (C₁₋₈)alkylsulfinyl, (C₁₋₈)alkylsulfinyl(C₁₋₈)alkyl, (C₁₋₈)alkylsulfonyl, (C₁₋₈)alkylsulfonyl(C₁₋₈)alkyl, amino, (C₁₋₈)alkylamino, di(C₁₋₈)alkylamino with two identical or different (C₁₋₈)alkyl moieties, amino(C₁₋₈)alkyl, (C₁₋₈)alkylamino(C₁₋₈)alkyl, di(C₁₋₈)alkylamino(C₁₋₈)alkyl with two identical or different (C₁₋₈)alkyl moieties in the di(C₁₋₈)alkylamino moiety, amino(C₁₋₈)alkoxy, (C₁₋₈)alkylamino(C₁₋₈)alkoxy, di(C₁₋₈)alkylamino(C₁₋₈)alkoxy with two identical or different (C₁₋₈)alkyl moieties, formyl, (C₁₋₈)-alkylcarbonyl, formyloxy, (C₁₋₈)alkylcarbonyloxy, formyl(C₁₋₈)alkyl, (C₁₋₈)alkylcarbonyl(C₁₋₈)alkyl, formyl(C₁₋₈)alkoxy, (C₁₋₈)alkylcarbonyl(C₁₋₈)alkoxy, (C₁₋₈)alkoxycarbonyl, (C₁₋₈)alkoxycarbonyloxy, (C₁₋₈)alkoxycarbonyl(C₁₋₈)alkyl, (C₁₋₈)alkoxycarbonyl(C₁₋₈)alkoxy, -OCH₂O-, -C(=O)OCH₂-, -CH₂OC(=O)- and - CH=CHCH=CH-, the four last-mentioned optional substituents in each case being attached to two adjacent ring carbon atoms of the said moiety.
- R¹: particular preferably represents hydrogen, a (C₁-C₈)alkyl group, or an aryl group, wherein said aryl group is unsubstituted,
wherein said alkyl group is unsubstituted, or mono-substituted, the optional substituent being selected from (C₂₋₄)alkenyl, (C₂₋₄)alkynyl, (C₁₋₈)alkoxy, phenyl, naphthyl.
- R¹: very preferably represents hydrogen.
- R²: preferably represents an aryl group or a (C₃-C₈)cycloalkyl group or a heterocyclyl group with 3 to 10 ring atoms or a heteroaryl group with 3 to 10 ring atoms or a (C₁-C₈)alkyl group;
wherein said aryl group, cycloalkyl group, heterocyclyl group, heteroaryl group, is unsubstituted, mono-substituted, di-substituted or tetra- substituted, the optional substituent(s) being independently selected from the group consisting of halogen, cyano, nitro, carboxy, carbamyl, hydroxy, (C₁₋₈)alkyl, (C₁₋₈)alkyl substituted by halogen, (C₃₋₈)cycloalkyl, (C₃₋₈)cycloalkyl(C₁₋₈)alkyl, (C₃₋₈)cycloalkoxy, (C₃₋₈)cycloalkoxy(C₁₋₈)alkyl, (C₃₋₈)cycloalkyl(C₁₋₈)alkoxy, (C₃₋₈)cycloalkoxy(C₁₋₈)alkoxy, aryl, aryl(C₁₋₈)alkyl, aryloxy, aryloxy(C₁₋₈)alkyl, aryl(C₁₋₈)alkoxy, aryloxy(C₁₋₈)alkoxy, (C₁₋₈)alkoxy, (C₁₋₈)alkoxy(C₁₋₈)alkoxy, (C₁₋₈)alkoxy substituted by halogen, (C₁₋₈)alkoxy(C₁₋₈)alkyl, (C₁₋₈)alkylthio, (C₁₋₈)alkylthio(C₁₋₈)alkyl, (C₁₋₈)alkylsulfinyl, (C₁₋₈)alkylsulfinyl(C₁₋₈)alkyl, (C₁₋₈)alkylsulfonyl, (C₁₋₈)alkylsulfonyl(C₁₋₈)alkyl, amino, (C₁₋₈)alkylamino, di(C₁₋₈)alkylamino with two identical or different (C₁₋₈)alkyl moieties, amino(C₁₋₈)alkyl, (C₁₋₈)alkylamino(C₁₋₈)alkyl, di(C₁₋₈)alkylamino(C₁₋₈)alkyl with two identical or different (C₁₋₈)alkyl moieties in the di(C₁₋₈)alkylamino moiety, amino(C₁₋₈)alkoxy, (C₁₋₈)alkylamino(C₁₋₈)alkoxy, di(C₁₋₈)alkylamino(C₁₋₈)alkoxy with two identical or different (C₁₋₈)alkyl moieties, formyl, (C₁₋₈)alkylcarbonyl, formyloxy, (C₁₋₈)alkylcarbonyloxy, formyl(C₁₋₈)alkyl, (C₁₋₈)alkylcarbonyl(C₁₋₈)alkyl, formyl(C₁₋₈)alkoxy, (C₁₋₈)alkylcarbonyl(C₁₋₈)alkoxy, (C₁₋₈)alkoxycarbonyl, (C₁₋₈)alkoxycarbonyloxy, (C₁₋₈)alkoxycarbonyl(C₁₋₈)alkyl, (C₁₋₈)alkoxycarbonyl(C₁₋₈)alkoxy, -OCH₂O-,-C(=O)OCH₂-, -CH₂OC(=O)- and -CH=CHCH=CH-, the four last-mentioned optional substituents in each case being attached to two adjacent ring carbon atoms of the said moiety and
wherein said (C₁₋₈)alkyl group is unsubstituted or mono-, di-, tri or tetra-substituted, the optional substituent(s) on the said (C₁₋₈)alkyl moiety being independently selected from the group consisting of halogen, cyano, oxo, (C₁₋₈)alkoxy, (C₁₋₈)alkoxy(C₁₋₈)alkoxy, (C₁₋₈)alkylthio, (C₁₋₈)alkylsulfinyl, (C₁₋₈) alkylsulfonyl, (C₁₋₈)alkylcarbonyloxy, (C₁₋₈)alkoxycarbonyl and (C₁₋₈)alkoxy carbonyloxy.
- R²: particular preferably represents an aryl group or a (C₃-C₈)cycloalkyl group or a heteroaryl group with 5 or 6 ring atoms, or a heterocyclyl group with 5 or 6 ring atoms or a (C₁-C₈)alkyl group
which is unsubstituted or mono-, di-, tri- or tetra-substituted on the aryl group, the optional substituent(s) on said moiety being independently selected from the group, consisting of halogen, cyano, (C₁₋₈)alkyl, (C₁₋₈)alkyl substituted by halogen, nitro, (C₁₋₈)alkoxy, (C₁₋₈)alkoxy substituted by halogen, (C₁₋₈)alkylthio, formyloxy, (C₁₋₈)alkylcarbonyloxy;
which is unsubstituted or mono-, di-, tri- or tetra-substituted on the (C₃₋C₈)cycloalkyl group, the optional substituent(s) on said group being independently selected from the group, consisting of halogen, cyano, (C₁₋₈)alkyl, (C₁₋₈)alkyl substituted by halogen, nitro, (C₁₋₈)alkoxy, (C₁₋₈)alkoxy substituted by halogen, (C₁₋₈)alkylthio, formyloxy, (C₁₋₈)alkylcarbonyloxy;
which is unsubstituted or mono-, di-, tri- or tetra-substituted on the heteroaryl group, the optional substituent(s) on the said group being independently selected from the group, consisting of halogen, cyano, (C₁₋₈)alkyl, (C₁₋₈)alkyl substituted by halogen, nitro, (C₁₋₈)alkoxy, (C₁₋₈)alkoxy substituted by halogen, (C₁₋₈)alkylthio, formyloxy, (C₁₋₈)alkylcarbonyloxy; and whereby the heteroarylmoiety contains 1-3 nitrogen atoms or 0-2 nitrogen and one oxygen or sulfur atom;
which is unsubstituted or mono-, di-, tri- or tetra-substituted on the heterocyclyl group, the optional substituent(s) on the said group being independently selected from the group, consisting of halogen, cyano, (C₁₋₈)alkyl, (C₁₋₈)alkyl substituted by halogen, nitro, (C₁₋₈)alkoxy, (C₁₋₈)alkoxy substituted by halogen, (C₁₋₈)alkylthio, formyloxy, (C₁₋₈)alkylcarbonyloxy; and whereby the heterocyclylmoiety contains 1-4 nitrogen atoms or 0-2 nitrogen and one oxygen or sulfur atom;
which is unsubstituted in the (C₁-C₈)alkyl group.
- R²: very preferably represents phenyl or naphthyl, which is unsubstituted or substituted by one or two substituents on the phenyl or naphthyl group, the optional substituents on said group being independently selected from fluoro, chloro, bromo, methyl, ethyl, propyl, tert-butyl, trifluormethyl, methoxy, ethoxy and trifluormethoxy.
- R²: preferably does not represent the CoA-residue. Specifically, in compounds of formula (I), where R¹ represents hydrogen, R² does not represent the CoA-residue, thereby excluding the compounds of Chang et al, discussed above.
However, in compounds of formula (I), where R¹ represents a substituent different from hydrogen, R² may also include the meaning of the CoA-residue.

In a further embodiment, the compound of formula (I) is selected from the following list of compounds:

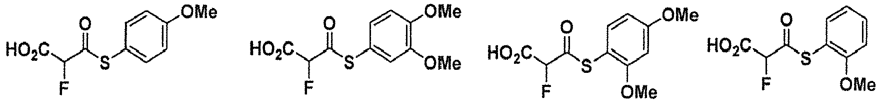

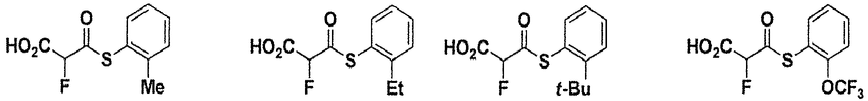

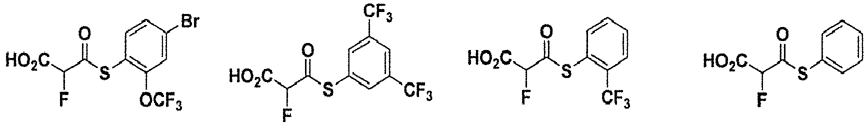

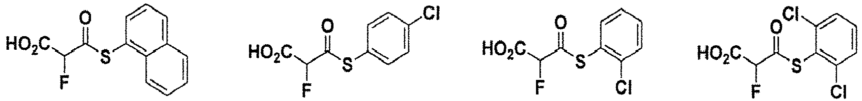

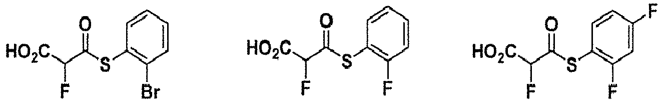

In a second aspect, the invention relates to methods for manufacturing a compound of formula (I) as described herein.

In a first embodiment, compounds of formula (I) are obtainable according to the processes which are summarized by the following scheme.

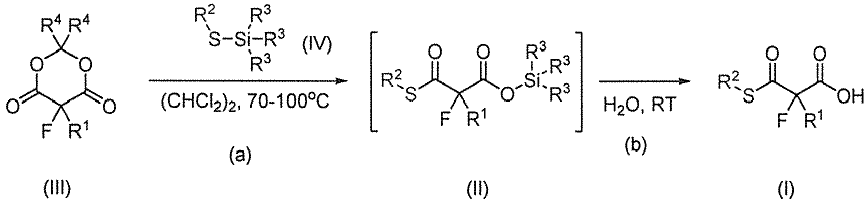

Generally, the compounds of formula (I) prepared via reaction of Silyl protected thiols (IV) with fluoromeldrum acid (III) in 1,1,2,2-tetrachloroethane at elevated temperature, preferably at 70-100°C (step (a)), followed by hydrolysis (step (b)). This aspect of the invention shall be explained in further detail below:
Step (a): Reactions according to step (a) are known per se, but not yet applied to the specific starting materials as defined herein. Reaction conditions may be determined by the skilled person. Step (a) optionally takes place in the presence of a reaction aid;
Step (a) is typically performed at temperatures between 50 - 150°C, preferably 70 - 100°C, and typically run for 0.1 -10 hrs, preferably 1 - 5 hrs. Step (a) preferably takes place in the presence of a diluent, such as halogenated solvents, e.g.
pentachloroethane or tetrachloroethane. Tetrachloroethane was found particularly suitable for obtaining good conversion rates and selectivity. The intermediates of formula (II) may or may not be isolated. Typically, no isolation takes place.

Without being bound to theory, the following considerations may help determining appropriate reaction conditions: (i) Due to electronic effect of the fluorine, structure (III) is much less reactive to nucleophilic opening than the non-fluorinated Meldrum acid, thus it requires much harsher conditions to be opened. (ii) At the same time the presence of the fluorine makes the intermediate structure (II) (but without the silyl protection) much more prone to undesired decarboxylation. In consequence all published conditions for this type of reaction for analogous substrates failed to provide compound I. The combination of a suitable diluent (such as the quite acidic, apolar, high boiling solvent TCE, activated the carbonyls of compound (III) and enabled mild reaction conditions) together with silylated nucleophiles (for the in situ silyl protection of the intermediate (II) that saved the malonate from decarboxylation) allowed performing the reaction step (a) with good yield and selectivity.

Step (b): reactions according to step (b) are known per se, but not yet applied to the specific starting materials as defined herein. Reaction conditions may be determined by the skilled person. Step (b) is typically performed at temperatures between 0 - 30°C and typically run for 0.01 - 5 hrs, preferably 0.01 - 1 hrs.

In one embodiment the invention provides for the manufacturing of a compound of formula (I) as described herein comprising the steps of:
(a) reacting a compound of formula (III) 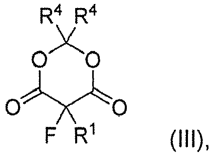 wherein R¹ is as defined herein and R⁴ independently represents a hydrogen, (C₁-C₈)alkyl group, or both R⁴ together with the carbon atom attached to represent (C₃-C₈)cycloalkyl group,
   with a compound of formula (IV) 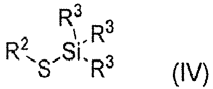 wherein R² is as defined herein, and R³ represents, independent from each other, a (C₁-C₈)alkyl group, a (C₁-C₈)alkoxy group, an aryl group, an aryl substituted by (C₁-C₈)alkyl; and
(b) hydrolysing the thus obtained compound.

The above method is considered advantageous, as it is comprises scalable reaction steps and as the starting materials are readily available.

The reactions can be effected according to conventional methods, for example as described in the Examples. The working-up of the reaction mixtures and the purification of the compounds thus obtainable may be carried out in accordance with known procedures. The starting materials of the formulae (III) - (IV) are known or may be prepared according to conventional procedures starting from known compounds, for example as described in the Examples. Selected starting materials and intermediates of the formulae (II) - (IV) are novel and subject of the present invention.

The invention thus provides for a compound of formula (II) 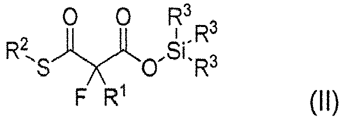 wherein
- R¹: is as defined herein;
- R²: is as defined herein; and
- R³: represents, independent from each other, a (C₁-C₈)alkyl group, a (C₁-C₈)alkoxy group, an aryl group, an aryl substituted by (C₁-C₈)alkyl.
- R³: preferably represents independent from each other methyl, ethyl, propyl, iso-propyl, butyl, tert-butyl, phenyl.
- R³: particularly preferably represents methyl.

In compounds of formula (II), R1 preferably represents hydrogen.

Compounds of formula (III) are obtainable according to the processes, which are summarized by the following scheme.

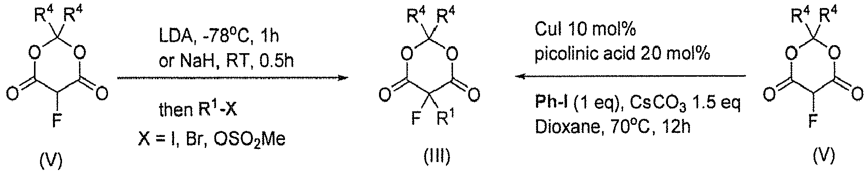

Compound (V) is deprotonated (e.g. with Lithium diisopropylamide (LDA) or sodium hydride (NaH)) followed by trapping of the resulting carbanion with a variety of alkylating agents of the structure R¹-X, where X is a good leaving group (e.g. I, Br, Cl, mesyl (CH₃SO₃-), tosyl (4-MeC₆H₄SO₃-)) attached to an sp³-hybridized carbon atom. This method is generally applicable to a wide variety of aliphatic groups. Few examples of common suitable alkylating agents would be Methyl iodide (Me-I), Benzyl bromide (PhCH₂Br) or Allyl bromide (CH₂=CH-CH₂Br), iso-propyl iodide ((CH₃)₂CHI), methoxymethyl chloride (CH₃OCH₂Cl).

Aromatic substituents (e.g. Ph) may also be introduced via Copper or Palladium-catalyzed cross coupling reactions, as known in the field.

Compounds of formula (IV) are obtainable according to the processes, which are summarized by the following scheme.

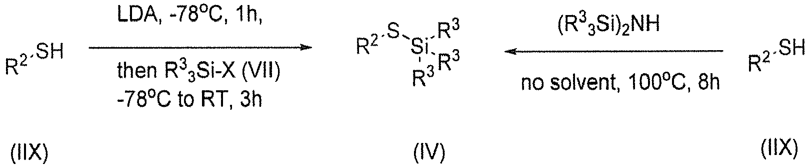

The silyl-protected thiols of formula (IV) were obtained either by trapping of the deprotonated thiols with TMSCI (trimethylsilyl chloride) or via thermal reaction of thiols with HMDS (hexamethyldisilazane). Other silyl groups that may be easily introduced using the respective starting materials, e.g. by using the trimethylsilyl-, triethylsilyl-, triisopropylsilyl-, tert-butyldimethylsilyl-, phenyldimethylsilyl-, triphenylsilyl-, or tertbutyldiphenylsilyl- derivatives.

In a second embodiment, compounds of formula (I) are obtainable according to the processes which are summarized by the following scheme.

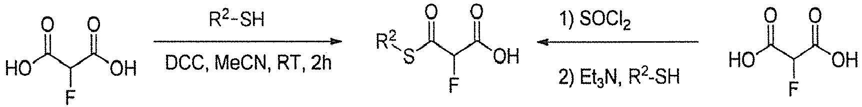

In this embodiment, a compound of formula (I) is obtained by coupling fluoromalonic acid with a thiol using a reaction aid. Suitable reaction aids include carbodiimide coupling reagents, like e.g. DCC, EDC or a chlorinating reagents like e.g. POCl₃, SOCl₂ and a base. This embodiment is particularly suitable for unsubstituted fluoromalonates (R¹ = H), thus no R¹ shown in the scheme. This route is further suitable for compounds of formula (I), where R¹ = halogen, using the respective starting material (not shown in the above scheme).

In a third aspect, the invention relates to the use of fluoromalonyl halfthioesters as described herein, as nucleophiles, particularly as masked fluoroacetates. This aspect of the invention shall be explained in further detail below:

In one embodiment, the inventive compounds of formula (I) may be used as a reagent in Aldol reactions, particularly in enantioselective Aldol reactions.

The compounds of formula (I) have nucleophilic properties and are useful reactants in nucleophilic addition reactions and nucleophilic substitution reactions. Since the reactions occur decarboxylatively, the CO₂ molecule is removed in the reaction. Compounds of formula (I) are equivalent to fluorothioacetate enolates, and thus masked fluoroacetates.

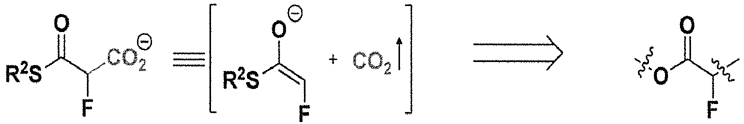

The invention thus provides for the use of the compounds of formula (I) in nucleophilic addition reactions and nucleophilic substitution reactions.

In one embodiment, the invention provides for the use of a compound of formula (I) in aldol reactions, i.e. the addition to aldehydes. The products of such reactions are suitable as precursors for fluorinated polyketides, or carbohydrates thus of high pharmaceutical importance. A specific embodiment illustrates this use:

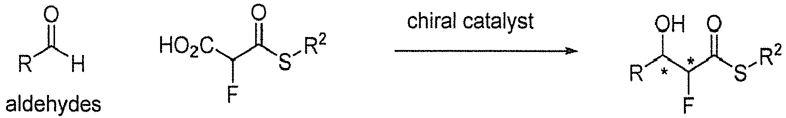

The reaction was exemplified using a pair of pseudoenantiomeric cinchona-alkaloid derived bifunctional catalysts: epi-Quinidine urea (eQDU) and epi-Quinine urea (eQNU) catalysts. The eQDU and eQNU catalysts achieved a series of (2S, 3S)- and (2R, 3*R*)-configured β-hydroxy-α-fluorothioesters respectively with enantioselectivities up to 99.5%ee, and diastereoselectivities up to 13:1.

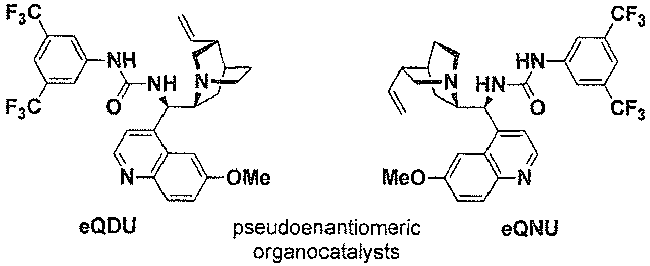

In one further embodiment, the invention provides for the use of a compound of formula (I) in conjugate addition to nitroolefins. The products of such reactions are suitable as precursors for fluorinated GABA (y-aminobutyric acid) derivatives and thus of high pharmaceutical importance. A specific embodiment illustrates this use:

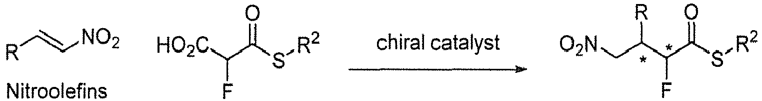

In one further embodiment, the invention provides for the use of a compound of formula (I) in Michael reactions, i.e. conjugate additions to α,β-unsaturated aldehydes, ketones and esters. The products of such reactions are suitable as fluorinated synthons of high synthetic value and pharmaceutical importance. A specific embodiment illustrates this use:

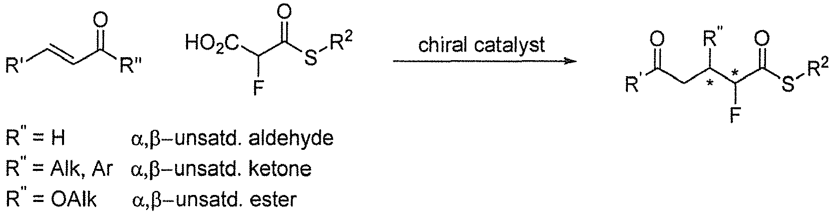

In one further embodiment, the invention provides for the use of a compound of formula (I) in Mannich reactions, i.e. additions to imines. The products of such reactions are fluorinated β-aminoacids, suitable as precursors for fluorinated β-peptides and thus of high pharmaceutical importance. A specific embodiment illustrates this use:

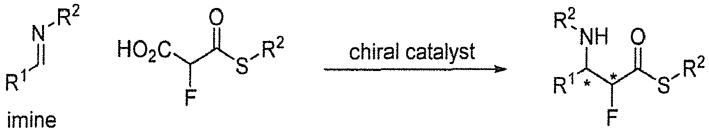

In one further embodiment, the invention provides for the use of a compound of formula (I) in alkylation reactions, i.e. nucleophilic substitution of alkyl halides (or alkyl sulfonates). The products of such reactions are suitable as precursors for fluorinated thioesters and thus fluorinated synhons of high synthetic value and pharmaceutical importance. A specific embodiment illustrates this use:

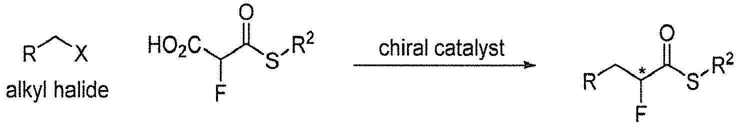

The above named, non-limiting reactions are known to the skilled person. Thus, the skilled person is in a position to identify suitable reaction conditions, dilutents and reaction aids. The invention thus provides for the use in any of the above reactions, wherein a diluent and / or a reaction aid is present.

The above named, non-limiting reactions are known to result in compounds having stereogenic centers and may thus be stereoselective (i.e. providing diastereomers or enantiomers). In a further embodiment, the invention provides for the use in any of the above reactions, which is diastereoselective, particularly enantioselective. In this embodiment, a chiral catalyst may be present.

In a further embodiment, the invention relates to the use of a compound of formula (I) or (II) in the manufacture of 2-fluoro-3-hydroxythioesters.

To further illustrate the invention, the following **examples** are provided. These examples are provided with no intend to limit the scope of the invention.

### A. General Aspects

Reaction mixtures were stirred magnetically. For all conjugate addition reactions, dry solvents were used under positive nitrogen pressure (balloon or Schlenk line).

All reagents and solvents were purchased at highest commercial quality and used as received. Anhydrous solvents were obtained from a purification column composed of activated alumina (A-2). All literature known compounds were characterized by comparison of at least two pieces of spectral data with that reported in the literature. Hydoxymethylene Meldrum's acid was synthesized according to Bihlmayer et al., Monatsh. Chem. 1967, 98, 564-578. Catalysts were synthesized according to McCooey, Angew. Chem. Int. Ed. 2005, 44, 6367-6370; or bought.

Yields refer to spectroscopically pure compounds unless otherwise stated. Rf: Flash chromatography was performed on silica gel (Merck Kieselgel 60 F₂₅₄ 230-400 mesh). TLC was performed on silica-backed silica plates (0.2 mm, 60 F254), which were developed using standard visualising agents: UV fluorescence (254 & 366 nm), iodine, ninhydrin / Δ, potassium permanganate / Δ. IR: Recorded on neat compounds using a Varian (Scimitar 800) FT-IR spectrometer. Only strong and selected absorbances (vₘₐₓ) are reported. 1H NMR: Spectra were recorded on Bruker NMR spectrometers (Avance 250, Avance 300, Avance 400 or DPX 400). Chemical shifts (δ_{H}) are quoted in parts per million (ppm) and referenced to the appropriate NMR solvent peak(s). ¹³C NMR: Spectra were recorded on Bruker NMR spectrometers (Avance 250, Avance 300, Avance 400 or DPX 400). Chemical shifts (δ_{c}) are quoted in ppm, referenced to the appropriate solvent peak(s). DEPT, COSY, HSQC and HMBC were used where necessary in assigning NMR spectra. m/z: Low resolution mass spectra were recorded on a Bruker Esquire 3000 Plus, with only major peaks being reported. HRMS: High resolution mass spectra were recorded on a Bruker Daltonics maXis apparatus. X-Ray: Full data is listed with the corresponding compounds and as cif files. LC-MS were performed on a Dionex Ultimate 3000 HPLC (Reprosil Gold)/Bruker amazon speed MS system. Chiral HPLC: Normal phase HPLC analyses were performed on an analytical HPLC with a diode array detector SPD-M10A from Shimadzu or on an Dionex UltiMate 3000 HPLC system (Thermo-Fisher) using Chiracel columns (AD-H, AS-H, OD-H) (250 mm x 4.6 mm) from Daicel under the reported conditions.

### B. Starting materials

### 5-(Ethoxymethylene)-2,2-dimethyl-1,3-dioxan-4,6-dione:

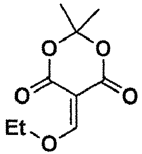

A mixture of Meldrum's acid (2,2-dimethyl-1,3-dioxan-4,6-dione, 40 g, 0.28 mol) and triethylorthoformate (134 g, 0.91 mol) was stirred at 85 °C for 4 h. Then the solvent was evaporated in vacuo to obtain the crude product as viscous yellow oil. The product was triturated with chloroform (20 mL) and the precipitate was filtered off and dried under vacuum to afford 5-(ethoxymethylene)-2,2-dimethyl-1,3-dioxan-4,6-dione as yellowish powder (42.6g, 76%).

### 5-(Hydroxymethylene)-2,2-dimethyl-1,3-dioxan-4,6-dione:

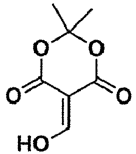

5-(ethoxymethylene)-2,2-dimethyl-1,3-dioxan-4,6-dione (13.9 g, 69.5 mmol) was vigorously stirred for 1 h in a solution of HCl 2 N (120 mL). The suspension was then diluted with brine and the water phase extracted with diethyl ether (3×100 mL). The organic phase was washed with brine (1×100 mL), dried over Na₂SO₄ and the solvent removed in vacuo to give the product as a yellow solid (8.26 g, 69%).

### 5-Fluoro-2,2-dimethyl-1,3-dioxane-4,6-dione (Fluoro-Meldrum's acid):

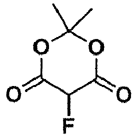

A dry, nitrogen-flushed, 3-necked round-bottom flask equipped with a thermometer, septum and a nitrogen inlet was charged with 8.26g (48 mmol) of 5-(hydroxymethylene)-2,2-dimethyl-1,3-dioxane-4,6-dione and 350 mL of dry acetonitrile. The mixture was cooled to -40 °C and 17.0g (48mmol) of Selectfluor^{®} was added in one portion. The mixture was vigorously stirred at -40 °C for 1 h, then allowed to reach RT over 3h and stirred for additional 2h at this temperature. The mixture was diluted with 200 mL of dichloromethane, 150 mL of 1N HCl and 100 mL of brine. The aqueous layer was extracted 3x with dichloromethane. Combined organic layers were dried over Na₂SO₄, filtered and concentrated to give crude F-Meld 7.2g. The product was recrystallized from dichloromethane (50 mL) to give Fluoro-Meldrum's acid as a colourless powder (5.90 g, 76%).

### Trimethylsilyl-protection of the thiols:

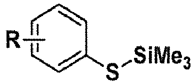

The thiols compatibile with butyllithium were TMS-protected according to the procedure A, the rest was prepared following procedure B:

**A:** To a solution of 4-Methoxybenzenethiol (5 mL, 40.7 mmol) in dry diethyl ether (50 mL) was added 1.6 M *n*-butyl lithium in hexane 28 mL (44.8 mmol) dropwise at -78 °C. The ice-bath was removed and the mixture was allowed to warm up to 0 °C, then it was cooled again to -78 °C and chlorotrimethylsilane 5.7 mL (44.9 mmol) was added dropwise. The reaction mixture was stirred at room temperature for 4 h, the solvent was evaporated, hexane (20 mL) was added and the precipitated solid was filtered off. The organic layer was evaporated and the residue was purified by distillation under reduced pressure to give the product as colorless oil (7.69 g, 89%). bp: 59 °C/0.1 mbar.

**B:** A neat mixture of 2-bromobenzenethiol 6 mL (50 mmol) and hexamethyldisilazane (1 eq) was heated at 100 °C for 8 h in a sealed tube. After cooling down the mixture was concentrated and the residue was distilled under reduced pressure to give the product as colorless oil (9.32 g, 71%). bp: 61 °C/0.07 mbar.

### C. Fluoromalonic acid halfthioesters (FMAHT):

FMAHTs were prepared according to the following representative procedure using tetrachloroethane as a solvent and the respective starting materials of formula (III) and (IV).

### 2-Fluoro-3-((4-methoxyphenyl)thio)-3-oxopropanoic acid:

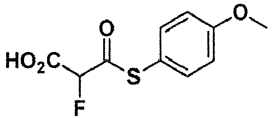

Fluoro-Meldrum's acid 1.75 g (10.8 mmol) was covered with 7 mL of tetrachloroethane, and ((4-methoxyphenyl)thio)- trimethylsilane 2.75 g (12.9 mmol) was added. The mixture was stirred at 80 °C for 5 h in a sealed tube. After cooling down, the mixture was diluted with dichloromethane (80 mL), saturated NaHCO₃ aq. (10 mL) and water (10 mL) and thoroughly shaken until gas evolution stopped. The aqueous layer was extracted with dichloromethane (4× 50 mL). Combined organic layers were discarded and the aqueous layer was acidified with cold 1 N HCl (80 mL) and extracted with dichloromethane (3x 50 mL). Combined organic layers were dried over Na₂SO₄, filtered, concentrated and dried under high vacuum to afford 2-Fluoro-3-((4-methoxyphenyl)thio)-3-oxopropanoic acid as a yellowish powder (2.24g, 85%).
**¹H** NMR [(400 MHz, CDCl₃/acetone-d₆ (5:1)] δ = 3.68 (s, 3H), 5.32 (d, *J* = 48.7 Hz, 1 H), 6.81-6.83, 7.17-7.20 (2m, 2×2H), 8.27 (br s, 1 H) ppm. **¹³C NMR** [101 MHz, CDCl₃. /acetone-d₆ (5:1)] δ = 55.2, 90.6 (d, *J =* 200.1 Hz), 115.0, 115.2 (d, *J =* 4.8 Hz), 136.2, 161.0, 163.9 *(d, J =* 24.2 Hz), 191.3 (d, *J* = 27.8 Hz) ppm. **¹⁹F NMR** [376 MHz, CDCl₃. /acetone-d₆ (5:1)] δ = -186.50 (d, *J* = 48.7 Hz) ppm. **IR** (neat): ṽ = 3506, 2946, 2841, 1744, 1699, 1592, 1495, 1292, 1249, 1176, 1109, 1070, 1025, 829, 799, 678 cm⁻¹. HRMS (ESI): C₁₀H₉FO₄S calcd. [M+Na]⁺ = 267.0098, found: 267.0100.

### 3-((3,4-Dimethoxyphenyl)thio)-2-fluoro-3-oxopropanoic acid:

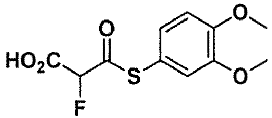

**¹H NMR** (300 MHz, CDCl₃) δ = 3.89, 3.91 (2s, 2×3H), 5.48 (d, *J* = 48.1 Hz, 1 H), 6.90-6.95, 7.01-7.05 (2m, 2H, 1 H), 7.27 (br s, 1 H) ppm. **¹⁹F NMR** (282 MHz, CDCl₃) δ = -191.2 (d, *J* = 48.1 Hz) ppm. **IR** (neat): ṽ = 3506, 2941, 2842, 1751, 1701, 1587, 1504, 1465, 1441, 1402, 1326, 1255, 1230, 1180, 1139, 1104, 1020, 879, 807, 766, 683 cm⁻¹. **HRMS** (ESI): C₁₁H₁₁FO₅S calcd. [M+H]⁺ = 275.0384, found: 275.0390.

### 3-((2,4-Dimethoxyphenyl)thio)-2-fluoro-3-oxopropanoic acid:

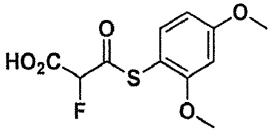

**¹H** NMR (400 MHz, CDCl₃) δ = 3.73, 3.75 (2s, 2x3H), 5.43 (d, *J* = 48.7 Hz, 1H), 6.88-6.97 (m, 3H), 9.77 (br s, 1H) ppm. **¹³C NMR** (101 MHz, CDCl₃) δ = 56.0, 56.7, 90.7 (d, *J* = 201.1 Hz), 112.9, 113.6 (d, *J* = 4.7 Hz), 117.9, 121.5, 153.7, 153.9, 164.5 (d, *J* = 24.2 Hz), 189.7 (d, *J* = 27.9 Hz) ppm. **¹⁹F NMR** (282 MHz, CDCl₃) δ = -191.4 (d, *J* = 48.7 Hz) ppm. **IR** (neat): ṽ = 3505, 3006, 2947, 2913, 2839, 1750, 1702, 1492, 1465, 1439, 1273, 1220, 1182, 1114, 1038, 872, 811, 737, 680 cm⁻¹. **HRMS** (ESI): C₁₁H₁₁FO₅S calcd. [M+H]⁺ = 275.0384, found: 275.0386.

### 2-Fluoro-3-((2-methoxyphenyl)thio)-3-oxopropanoic acid:

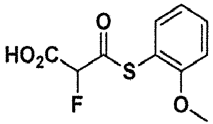

**¹H NMR** (300 MHz, CDCl₃) δ = 3.83 (s, 3H), 5.46 (d, *J* = 48.1 Hz, 1H), 6.97-7.03, 7.37-7.49 (2m, 2×2H) ppm. **¹³C** NMR (75 MHz, CDCl₃) δ = 56.3, 90.6 (d, *J* = 201.5 Hz), 111.8, 112.5, 121.3, 132.5, 136.6, 159.2, 165.7 (d, *J* = 23.6 Hz), 190.0 (d, *J* = 27.7 Hz) ppm. **¹⁹F NMR** (282 MHz, CDCl₃) δ = -190.5 (d, *J* = 48.1 Hz) ppm. **IR** (neat): ṽ = 3527, 2946, 2842, 1742, 1702, 1585, 1480, 1435, 1277, 1250, 1184, 1114, 1062, 1020, 798, 755, 688 cm⁻¹. **HRMS** (ESI): C₁₀H₉FO₄S calcd. [M+Na]⁺ = 267.0098, found: 267.0101.

### 2-Fluoro-3-oxo-3-((2-(trifluoromethoxy)phenyl)thio)propanoic acid:

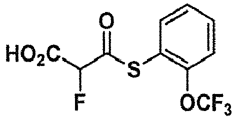

**¹H NMR** (400 MHz, CDCl₃) δ = 5.50 (d, *J =* 48.2 Hz, 1 H), 7.35-7.42, 7.52-7.57 (2m, 2×2H), 8.13 (br s, 1H) ppm. **¹³C NMR** (101 MHz, CDCl₃) δ = 90.4 (d, *J* = 203.4 Hz), 118.4 (d, *J =* 5.3 Hz), 120.4 (d, *J* = 259.4 Hz), 121.5 (d, *J* = 1.6 Hz), 127.7, 132.7, 137.5, 149.9, 166.8 (d, *J* = 24.2 Hz), 188.9 (d, *J* = 28.8 Hz) ppm. **¹⁹F NMR** (376 MHz, CDCl₃) δ =-57.5, -192.6 (d, *J =* 48.2 Hz) ppm. **IR** (neat): ṽ = 3538, 2954, 1750, 1711, 1589, 1476, 1447, 1250, 1208, 1175, 1117, 1063, 981, 925, 869, 819, 766, 739, 673 cm⁻¹. **HRMS** (EI): Chemical Formula: C₁₀H₆F₄O₄S calcd. [M-CO₂]⁺ = 254.0025 found: 254.0019.

### 2-Fluoro-3-oxo-3-(phenylthio)propanoic acid

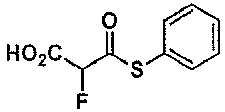

**¹H NMR** (400 MHz, CDCl₃) δ = 5.45 (d, *J* = 48.5 Hz, 1 H), 7.41-7.45 (m, 5H), 10.22 (br s, 1 H) ppm. **¹³C NMR** (101 MHz, CDCl₃) δ = 90.7 (d, *J* = 201.6 Hz), 124.9 (d, *J* = 4.7 Hz), 129.6, 130.2, 134.8, 164.8 (d, *J =* 24.2 Hz), 190.9 (d, *J =* 28.0 Hz) ppm. **¹⁹F NMR** (376 MHz, CDCl₃) δ = -191.74 (d, *J =* 48.5 Hz) ppm. **IR** (neat): ṽ = 3522, 3062, 2954, 1744, 1701, 1628, 1478, 1442, 1327, 1265, 1214, 1115, 1069, 1024, 748, 706, 689 cm⁻¹. **HRMS** (EI): Chemical Formula: C₉H₇FO₃S calcd. [M]⁺ = 214.0100 found: 214.0095.

### 2-Fluoro-3-(naphthalen-1-ylthio)-3-oxopropanoic acid:

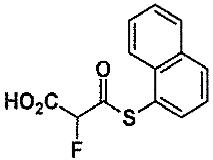

**¹H NMR** [(400 MHz, CDCl₃/acetone-d₆ (5:1)] δ = 5.50 (d, *J =* 48.7 Hz, 1 H), 7.45-7.54, 7.67-7.70, 7.85-7.87, 7.94-7.96, 8.07-8.09 (5m, 3H, 4×1H), 8.56 (br s, 1H) ppm. **¹³C NMR** [101 MHz, CDCl₃/acetone-d₆ (5:1)] δ = 90.9 (d, *J* = 200.9 Hz), 122.3 (d, *J* = 4.3 Hz), 124.9, 125.7, 126.7, 127.6, 128.8, 131.6, 134.2 (d, *J* = 5.2 Hz), 135.5, 164.3 (d, *J* = 23.7 Hz), 190.4 (d, *J* = 27.1 Hz) ppm. **¹⁹F NMR** [376 MHz, CDCl₃/acetone-d₆ (5:1)] δ = -190.8 (d, *J* = 48.7 Hz) ppm. **IR** (neat): ṽ = 3505, 3057, 2952, 1742, 1701, 1591, 1504, 1381, 1338, 1256, 1205, 1113, 1073, 971, 913, 865, 798, 770, 669 cm⁻¹. **HRMS** (ESI): Chemical Formula: C₁₃H₉FO₃S calcd. [M+H]⁺ = 265.0329, found: 265.0323.

### 2-Fluoro-3-oxo-3-(o-tolylthio)propanoic acid:

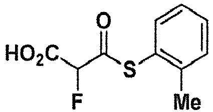

**¹H NMR** (300 MHz, CDCl₃) δ = 2.35 (s, 3H), 5.48 (d, *J* = 47.9 Hz, 1 H), 7.23-7.43 (m, 4H), 8.29 (br s, 1 H) ppm. **¹⁹F NMR** (282 MHz, CDCl₃) δ = -190.5 (d, *J* = 47.9 Hz) ppm. **IR** (neat): ṽ = 3504, 3059, 2964, 2924, 2854, 1749, 1701, 1653, 1589, 1478, 1461, 1275, 1042, 747, 708, 656 cm⁻¹. **HRMS** (EI): Chemical Formula: C₁₀H₉FO₃S calcd. [M]⁺ = 228.0256 found: 228.0254.

### 3-((2-Ethylphenyl)thio)-2-fluoro-3-oxopropanoic acid:

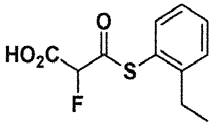

**¹H NMR** (300 MHz, CDCl₃) δ = 1.19 (t, *J* = 7.5 Hz, 3H), 2.70 (q, *J* = 7.5 Hz, 2H), 5.49 (d, *J* = 48.0 Hz, 1 H), 7.24-7.46 (m, 4H), 8.36 (br s, 1H) ppm. **¹⁹F NMR** (282 MHz, CDCl₃) δ = -190.5 (d, *J* = 48.0 Hz) ppm. **HRMS** (ESI): C₁₁H₁₁FO₃S calcd. [M+H]⁺ = 243.0486, found: 243.0489.

### 3-((2-(tert-Butyl)phenyl)thio)-2-fluoro-3-oxopropanoic acid:

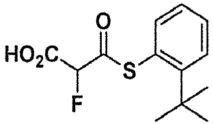

**¹H** NMR (300 MHz, CDCl₃) δ = 1.43 (s, 9H), 5.48 (d, *J* = 48.0 Hz, 1 H), 7.24-7.44, 7.53-7.56 (2m, 3H, 1 H) ppm. **¹⁹F NMR** (282 MHz, CDCl₃) δ = -190.4 (d, *J =* 48.0 Hz) ppm.

### 2-Fluoro-3-((2,6-Dimethylphenyl)thio)-3-oxopropanoic acid:

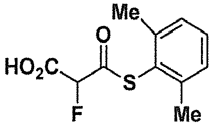

**¹H NMR** (400 MHz, CDCl₃) δ = 2.37 (s, 6H), 5.51 (d, *J* = 48.3 Hz, 1H), 7.20-7.22, 7.29-7.33 (2m, 2H, 1H), 10.24 (br s, 1H) ppm. **¹³C NMR** (101 MHz, CDCl₃) δ = 21.6, 90.44 (d, *J* = 203.2 Hz), 123.8 (d, *J* = 3.7 Hz), 128.8, 130.8, 143.2 167.77 (d, *J =* 23.9 Hz), 189.52 (d, *J =* 27.3 Hz) ppm. **¹⁹F NMR** (376 MHz, CDCl₃) δ = -191.3 (d, *J =* 48.3 Hz) ppm. IR (neat): ṽ = 3527, 3058, 2957, 2926, 1742, 1686, 1584, 1463, 1439, 1379, 1253, 1214, 1111, 1077, 1047, 980, 912, 870, 774, 681 cm⁻¹. **HRMS** (ESI): C₁₁H₁₁FO₃S calcd. [M+H]⁺ = 243.0486, found: 243.0486.

### 2-Fluoro-3-oxo-3-((2-(trifluoromethyl)phenyl)thio)propanoic acid

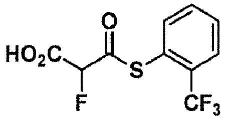

**¹H** NMR [(400 MHz, CDCl₃/acetone-d₆ (5:1)] δ = 5.38 (d, *J =* 48.6 Hz, 1 H), 7.47-7.56, 7.70-7.72 (2m, 3H, 1 H), 9.97 (br s, 1 H) ppm. **¹³C NMR** [101 MHz, CDCl₃/acetone-d₆ (5:1)] δ = 90.6 (d, *J* = 200.7 Hz), 122.9 (d, *J* = 273.7 Hz), 123.6 (d, *J* = 5.6 Hz), 127.3 (d, *J* = 5.7 Hz), 130.6, 132.5, 139.1, 163.4 *(d, J =* 24.0 Hz), 189.5 (d, *J* = 28.8 Hz) ppm. **¹⁹F NMR** [376 MHz, CDCl₃/acetone-d₆ (5:1)] δ = -55.5, -186.4 (d, *J* = 48.6 Hz) ppm. **IR** (neat): ṽ = 3538, 2957, 1750, 1709, 1593, 1440, 1313, 1290, 1267, 1177, 1115, 1074, 1033, 962, 913, 868, 768, 708, 680 cm⁻¹. **HRMS** (EI): Chemical Formula: C₁₀H₆F₄O₃S calcd. [M-CO₂]⁺ = 238.0075 found: 238.0073.

### 2-Fluoro-3-((4-chlorophenyl)thio)-3-oxopropanoic acid

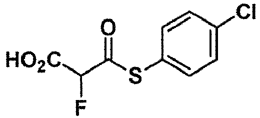

**¹H NMR** [(400 MHz, CDCl₃/acetone-d₆ (5:1)] δ = 5.41 (d, *J* = 48.4 Hz, 1 H), 7.30-7.37 (m, 4H), 8.76 (br s, 1 H) ppm. **¹³C NMR** [101 MHz, CDCl₃/acetone-d₆ (5:1)] δ = 90.6 (d, *J =* 200.7 Hz), 123.5 (d, *J* = 5.1 Hz), 129.6, 136.0, 136.4, 163.7 (d, *J* = 24.1 Hz), 190.3 (d, *J* = 28.4 Hz) ppm. **¹⁹F NMR** [376 MHz, CDCl₃/acetone-d₆ (5:1)] δ = -186.5 (d, *J* = 48.4 Hz) ppm. **IR** (neat): ṽ = 3505, 3091, 2950, 1741, 1697, 1638, 1574, 1474, 1422, 1390, 1327, 1266, 1209, 1112, 1093, 1079, 1013, 977, 910, 817, 747, 690 cm⁻¹. **HRMS** (EI): Chemical Formula: C₉H₆ClFO₃S calcd. [M-CO₂]⁺ = 203.9812 and 205.9782 found: 203.9805 and 205.9779.

### 2-Fluoro-3-((2-Bromophenyl)thio)-3-oxopropanoic acid:

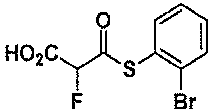

**¹H NMR** [(400 MHz, CDCl₃/acetone-d₆ (5:1)] δ = 5.44 (d, *J =* 48.5 Hz, 1 H), 7.24-7.35, 7.47-7.50, 7.64-7.67 (3m, 2H, 2×1H), 9.23 (br s, 1 H) ppm. **¹³C NMR** [101 MHz, CDCl₃/acetone-d₆ (5:1)] δ = 90.7 (d, *J* = 200.8 Hz), 126.9 (d, *J* = 5.4 Hz), 128.2, 129.6, 131.8, 133.8, 137.3, 163.60 (d, *J =* 24.1 Hz), 188.93 (d, *J =* 28.7 Hz) ppm. **¹⁹F NMR** [376 MHz, CDCl₃/acetone-d₆ (5:1)] δ = -191.7 (d, *J* = 48.5 Hz) ppm. **IR** (neat): ṽ = 3549, 3061, 2953, 1745, 1707, 1625, 1576, 1561, 1450, 1431, 1321, 1256, 1215, 1114, 1071, 1021, 754, 683 cm⁻¹. **HRMS** (ESI): Chemical Formula: C₉H₆BrFO₃S calcd. [M+Na]⁺ = 314.9097 and 316.9077, found: 314.9095 and 316.9067.

### 2-Fluoro-3-((2-chlorophenyl)thio)-3-oxopropanoic acid

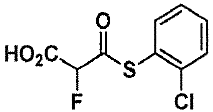

**¹H NMR** [(400 MHz, CDCl₃/acetone-d₆ (5:1)] δ = 5.45 (d, *J =* 48.5 Hz, 1 H), 7.22 (br s, 1 H), 7.28-7.32, 7.36-7.41, 7.48-7.52 (3m, 2×1H, 2H) ppm. **¹³C NMR** [101 MHz, CDCl₃/acetone-d₆ (5:1)] δ = 90.8 (d, *J =* 201.2 Hz), 124.7 (d, *J =* 5.3 Hz), 127.6, 130.6, 131.9, 137.3, 139.1, 164.0 (d, *J* = 24.2 Hz), 189.1 (d, *J* = 28.7 Hz) ppm. **¹⁹F NMR** [376 MHz, CDCl₃₋/acetone-d₆ (5:1)] δ = -191.6 (d, *J* = 48.9 Hz) ppm. **IR** (neat): ṽ = 3549, 3066, 2957, 1750, 1708, 1626, 1575, 1454, 1434, 1322, 1257, 1214, 1116, 1073, 1036, 982, 915, 868, 756, 684, 661 cm⁻¹. **HRMS** (EI): Chemical Formula: C₉H₆OFO₃S calcd. [M]⁺ = 247.9710 and 249.9681 found: 247.9713 and 249.9691.

### 2-Fluoro-3-((2-fluorophenyl)thio)-3-oxopropanoic acid

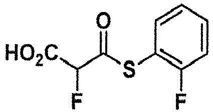

**¹H NMR** [(400 MHz, CDCl₃/acetone-d₆ (5:1)] δ = 5.41 (d, *J =* 48.4 Hz, 1H), 7.08-7.15, 7.32-7.42 (2m, 2x2H), 10.21 (brs, 1H) ppm. **¹³C NMR** [101 MHz, CDCl₃/acetone-d₆ (5:1)] δ = 90.7 (d, *J =* 200.5 Hz), 112.4 (dd, *J* = 18.7, 5.3 Hz), 116.3 (d, *J =* 22.4 Hz), 124.9 (d, *J* = 3.9 Hz), 132.8 (d, *J* = 8.2 Hz), 136.6, 162.2 (d, *J* = 250.7 Hz), 163.7 (d, *J* = 24.1 Hz), 188.9 (d, *J =* 29.0 Hz) ppm. **¹⁹F NMR** [376 MHz, CDCl₃/acetone-d₆ (5:1)] δ = -101.1, -186.4 (d, *J* = 48.4 Hz) ppm. **IR** (neat): ṽ = 3547, 3055, 2948, 1742, 1702, 1625, 1597, 1581, 1476, 1448, 1264, 1228, 1113, 1080, 1065, 974, 949, 826, 757, 681 cm⁻¹. **HRMS** (EI): Chemical Formula: C₉H₆F₂O₃S calcd. [M]⁺ = 232.0006 found: 231.9998.

### 2-Fluoro-3-((2,4-difluorophenyl)thio)-3-oxopropanoic acid

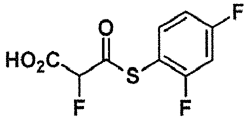

**¹H** NMR (400 MHz, CDCl₃) δ = 5.50 (d, *J* = 48.2 Hz, 1H), 5.90 (br s, 1 H), 6.95-7.01, 7.38-7.44 (2m, 2H, 1 H) ppm. **¹³C** NMR (101 MHz, CDCl₃) δ = 90.4 (d, *J* = 202.8 Hz), 105.5 z(t, *J* = 26.1 Hz), 107.9 (ddd, *J* = 18.9, 5.7, 4.2 Hz), 112.9 (dd, *J =* 22.0, 3.8 Hz), 137.8 (dd, *J* = 10.1, 1.8 Hz), 163.0 (dd, *J* = 253.6, 12.7 Hz), 165.1 (dd, *J* = 254.5, 11.5 Hz), 165.9 (d, *J* = 24.1 Hz), 189.2 (d, *J* = 29.0 Hz) ppm. **¹⁹F NMR** (376 MHz, CDCl₃) δ = - 100.8, -104.2, -192.70 (d, *J* = 48.2 Hz) ppm. **IR** (neat): ṽ = 3533, 3104, 2959, 1750, 1714, 1601, 1489, 1424, 1270, 1145, 1117, 1078, 968, 855, 814, 736, 685 cm⁻¹. **HRMS** (EI): Chemical Formula: C₉H₅F₃O₃S calcd. [M]⁺ = 249.9911 found: 249.9916.

### D. Aldol Reactions:

Procedure **A:** Used for electron deficient aromatic aldehydes: The catalyst (20 mol%), FMAHT (2 eq), and 4-DMAP (30 mol%) were dissolved in anhydrous THF (10mL/mmol) and cooled to 0°C. The aldehyde (1 eq, typically 0.2 mmol) was added and the reaction was stirred at 0°C for 3-5 days. After this time most of the THF was quickly removed in vacuo (bath temperature <30°C) and the mixture was transferred directly to the column (Merck Silica gel 10180 Grade, 50g/mmol) and eluted using hexane/dichloromethane/ether (1:1:0) → hexane/dichloromethane/ether (4:4:2). The tricomponent eluent was often beneficial for separation of the *anti*/*syn* diastereoisomers.

Procedure **B:** Used for all other substrates: The bifunctional cinchona alkaloid-derived catalyst (20 mol%) and FMAHT (2-3 eq), were dissolved in anhydrous THF (10mL/mmol) and cooled <10°C. The aldehyde (1 eq, typically 0.2 mmol) was added and the reaction was stirred at 10°C for 3-5 days. The same workup and purification as in the procedure A followed.

### (2S,3S)-S-(4-Methoxyphenyl) 2-fluoro-3-hydroxy-3-(4-nitrophenyl) propanethioate:

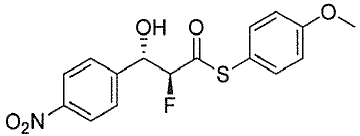

(81% yield, dr 5:1 *anti*/*syn*); *anti*-isomer: **¹H NMR** [400 MHz, C₆D₆/Acetone-*d*₆ (3:1)] δ = 3.31 (s, 3H), 5.11-5.29 (m, 3H), 6.69-6.72, 7.12-7.15, 7.39-7.41, 7.95-7.97 (4m, 4×2H) ppm. **¹³C NMR** [101 MHz, C₆D₆/Acetone-*d*₆ (3:1)] δ = 55.0, 73.4 (d, *J* = 20.7 Hz), 97.7 (d, *J* = 196.1 Hz), 115.4, 116.5 (d, *J =* 5.6 Hz), 123.4, 128.6, 136.6, 145.7 (d, *J =* 3.7 Hz), 148.2, 161.5, 196.1 (d, *J* = 28.2 Hz) ppm. **¹⁹F NMR** [377 MHz, C₆D₆/Acetone-*d*₆ (3:1)] δ = - 196.60 (dd, *J* = 48.3, 20.3 Hz) ppm. **IR** (neat): ṽ = 3512, 3114, 2944, 2915, 2841, 1693, 1593, 1575, 1521, 1496, 1464, 1347, 1293, 1251, 1176, 1109, 1078, 1028, 857, 829, 800, 723 cm⁻¹. **HRMS** (ESI): C₁₆H₁₄FNO₅S calcd. [M+Na]⁺ = 374.0469, found: 374.0468. 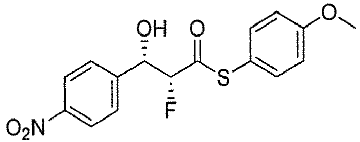 *syn*-isomer (2R, 3*S*): **¹H NMR** [400 MHz, C₆D₆/Acetone-*d*₆ (3:1)] δ = 3.30 (s, 3H), 4.90 (dd, *J* = 47.2, 2.4 Hz, 1 H), 5.04 (d, *J* = 6.2 Hz, 1 H), 5.28 (ddd, *J* = 26.7, 6.2, 2.4 Hz, 1H), 6.70-6.74, 7.27-7.31, 7.90-7.92 (3m, 2H, 4H, 2H) ppm. **¹³C NMR** [101 MHz, C₆D₆/Acetone-*d*₆ - (3:1)] δ = 55.0, 73.0 *(d, J =* 18.8 Hz), 97.9 (d, *J* = 198.1 Hz), 115.4, 117.0 *(d, J =* 5.9 Hz), 123.5, 136.8, 146.9 *(d, J =* 2.2 Hz), 148.0, 161.4, 196.5 (d, *J =* 29.7 Hz) ppm; one of the aromatic carbon atom signals is buried under the C₆D₆ signal. **¹⁹F NMR** [377 MHz, C₆D₆/Acetone-*d*₆ (3:1)] δ = -204.8 (dt, *J* = 47.2, 26.7 Hz) ppm. **IR** (neat): ṽ = 3495, 3114, 3008, 2925, 2852, 1693, 1593, 1520, 1496, 1463, 1348, 1294, 1252, 1176, 1106, 1028, 856, 829, 746, 720 cm⁻¹. **HRMS** (ESI): C₁₆H₁₄FNO₅S calcd. [M+Na]⁺ = 374.0469, found: 374.0472.
**HPLC:** Daicel Chiracel OD-H, 30% *i*-PrOH/hexane, 0.6 mL/min., 25°C, *anti* 99%ee, *syn* 97%ee, (t_{R} (1, minor *anti*-enantiomer) = 14.21 min, t_{R} (2, major *anti*-enantiomer) = 17.89 min, t_{R} (3, minor *syn*-enantiomer) = 37.18 min, t_{R} (4, major *syn*-enantiomer) = 71.92 min).

### (2S,3S)-S-(4-Methoxyphenyl) 3-(4-cyanophenyl)-2-fluoro-3-hydroxypropane-thioate:

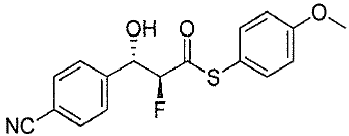

(84% yield, dr 5:1 *anti*/*syn*); **¹H NMR** [400 MHz, C₆D₆/Acetone-*d*₆ (3:1)] δ = 3.34 (s, 3H), 4.87-5.30 (m, 3H), 6.70-6.75, 7.10-7.13, 7.22-7.38 (3m, 2×2H, 4H) ppm. **¹³C NMR** [101 MHz, C₆D₆/Acetone-*d*₆ (3:1)] δ = 55.0, 73.6 (d, *J* = 20.6 Hz), 97.7 *(d, J =* 195.9 Hz), 112.4, 115.4, 116.6 (d, *J* = 5.7 Hz), 118.8, 128.5, 132.0, 136.7, 143.7, 161.5, 196.1 (d, *J =* 28.2 Hz) ppm. Visible signals of the *syn* diastereoisomer δ = 73.1 (d, *J =* 18.8 Hz), 132.1, 136.8 ppm. **¹⁹F NMR** [377 MHz, C₆D₆/Acetone-*d*₆ (3:1), ¹H-decoupled] δ = - 196.9 (*anti*-isomer), -204.8 (*syn*-isomer) ppm.
**IR** (neat): ṽ = 3463, 3005, 2930, 2840, 2231, 1693, 1593, 1574, 1495, 1463, 1441, 1408, 1292, 1251, 1176, 1097, 1080, 1029, 857, 828, 799, 773, 743, 656 cm⁻¹. **HRMS** (ESI): C₁₇H₁₄FNO₃S calcd. [M+Na]⁺ = 354.0571, found: 354.0571.
**HPLC:** Daicel Chiracel OD-H, 30% *i*-PrOH/hexane, 0.6 mL/min., 25°C, *anti* 99%ee, *syn* 98%ee, (t_{R} (1, minor *anti*-enantiomer) = 13.18 min, t_{R} (2, major *anti*-enantiomer) = 15.58 min, t_{R} (3, minor *syn*-enantiomer) = 29.10 min, t_{R} (4, major *syn*-enantiomer) = 57.81 min).

### (2S,3S)-S-(4-Methoxyphenyl) 3-([1,1'-biphenyl]-4-yl)-2-fluoro-3-hydroxypropane-thioate:

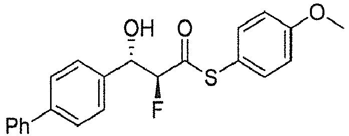

(46% yield, dr 6:1 *anti*/*syn*); *anti*-isomer: **¹H NMR** [400 MHz, C₆D₆/Acetone-*d*₆ (3:1)] δ = 3.25 (s, 3H), 4.50-4.52 (m, 1 H), 5.16-5.32 (m, 2H), 6.65-6.68, 7.10-7.22, 7.44-7.55 (3m, 2H, 5H, 6H) ppm. **¹³C**

NMR [101 MHz, C₆D₆/Acetone-*d*₆ (3:1)] δ = 54.9, 74.2 (d, *J* = 20.7 Hz), 97.9 (d, *J* = 195.3 Hz), 115.3, 116.9 (d, *J* = 5.6 Hz), 127.1, 127.4, 127.6, 128.4, 129.1, 136.7, 137.8 (d, *J* = 2.7 Hz), 141.2, 141.4, 161.3, 196.6 (d, *J =* 28.1 Hz) ppm. **¹⁹F NMR** [377 MHz, C₆D₆/Acetone-*d*₆ (3:1)] δ = -194.82 (dd, *J* = 49.2, 15.4 Hz) ppm. **IR** (neat): ṽ = 3461, 3056, 3031, 2940, 2838, 1691, 1593, 1574, 1495, 1461, 1440, 1408, 1292, 1250, 1175, 1096, 1078, 1030, 1008, 827, 800, 753, 698 cm⁻¹. **HRMS** (ESI): C₂₂H₁₉FO₃S calcd. [M+Na]⁺ = 405.0931, found: 405.0937. 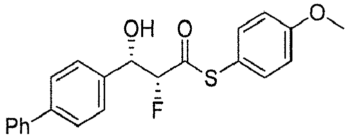 *syn*-isomer (2*R*, 3S): **¹H NMR** [400 MHz, C₆D₆/Acetone-*d*₆ (3:1)] δ = 3.33 (s, 3H), δ 4.81 (d, *J* = 6.1 Hz, 1 H), 5.05 (dd, *J* = 47.2, 2.6 Hz, 1 H), 5.36 (ddd, *J* = 26.8, 6.1, 2.6 Hz, 1H), 6.70-6.74, 7.12-7.30, 7.44-7.50 (3m, 2H, 6H, 5H) ppm. **¹³C NMR** [101 MHz, C₆D₆/Acetone-*d*₆ - (3:1)] δ = 55.0, 73.7 (d, *J =* 18.8 Hz), 98.5 (d, *J* = 197.1 Hz), 115.3, 117.3 (d, *J* = 5.7 Hz), 127.3, 127.4, 127.6, 127.7, 129.1, 136.8, 139.2 (d, *J =* 2.1 Hz), 141.1, 141.2, 161.3, 196.8 (d, *J* = 29.9 Hz) ppm. **¹⁹F NMR** [377 MHz, C₆D₆/Acetone-*d*₆ (3:1)] δ = - 203.6 (ddd, *J* = 47.2, 26.8, 5.0 Hz) ppm. **IR** (neat): ṽ = 3439, 3023, 3005, 2971, 2945, 2843, 1690, 1592, 1574, 1495, 1455, 1440, 1366, 1248, 1229, 1217, 1178, 1095, 1031, 898, 827, 696, 680 cm⁻¹. **HRMS** (ESI): C₂₂H₁₉FO₃S calcd. **[M+Na]⁺** = 405.0931, found: 405.0929.
**HPLC:** Daicel Chiracel OD-H, 30% *i*-PrOH/hexane, 0.6 mL/min., 25°C, *anti* >99%ee, *syn* 97%ee, (t_{R} (1, minor *anti*-enantiomer) = 14.30 min, t_{R} (2, major *anti*-enantiomer) _ 18.34 min, t_{R} (3, minor *syn*-enantiomer) = 25.81 min, t_{R} (4, major *syn*-enantiomer) = 35.33 min).

### (2S,3S)-S-(4-methoxyphenyl) 2-fluoro-3-hydroxy-3-(4-(trifluoromethyl)phenyl)-propanethioate:

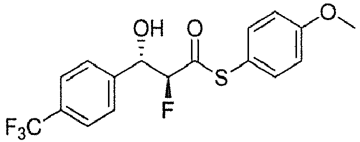

(62% yield, dr 6:1 *anti*/*syn*); **¹H NMR** [400 MHz, C₆D₆/Acetone-*d*₆(3:1)] δ = 3.33 (s, 3H), 4.92-5.34 (m, 3H), 6.69-6.75, 7.11-7.28, 7.43-7.49 (3m, 2×2H, 4H) ppm. ¹³C **NMR** [101 MHz, C₆D₆/Acetone-*d*₆ (3:1)] δ = 55.0, 73.6 (d, *J* = 20.7 Hz), 97.8 (d, *J* = 195.6 Hz), 115.3, 116.6 (d, *J*=5.7 Hz), 125.2 (q, *J* = 3.9 Hz), 127.7, 128.4, 130.2 (q, *J* = 32.6, 32.0 Hz), 136.7, 143.1, 161.4, 196.2 (d, *J* = 28.2 Hz) ppm. Visible signals of the *syn* diastereoisomer δ = 73.2 (d, *J* = 18.5 Hz), 98.1 (d, *J* = 197.5 Hz), 117.1 (d, *J* = 5.8 Hz), 136.8, 144.4, 196.64 (d, *J =* 29.9 Hz) ppm. ¹⁹**F NMR** [377 MHz, C₆D₆/Acetone-*d*₆(3:1)] δ = -62.4 (m, CF₃), -196.7 (m, *anti*-isomer), -204.9 (dddd, *J=* 45.7, 27.1, 12.1, 5.6 Hz, *syn*-isomer) ppm. **IR** (neat): ṽ = 3395, 3027, 2969, 2947, 2846, 1696, 1620, 1593, 1572, 1495, 1462, 1443, 1420, 1363, 1324, 1293, 1251, 1169, 1126, 1067, 1027, 858, 828, 810, 800, 776, 750, 735, 699 cm⁻¹. **HRMS** (ESI): C₁₇H₁₄F₄O₃S calcd. [M+Na]⁺ = 397.0492, found: 397.0499. HPLC: Daicel Chiracel OD-H, 25% i-PrOH/hexane, 1.0 mL/min., 25°C, *anti* 98%ee, *syn* 98%ee, (t_{R} (1, minor *anti*-enantiomer) = 6.73 min, t_{R} (2, major *anti*-enantiomer) = 7.98 min, t_{R} (3, minor *syn*-enantiomer) = 12.48 min, t_{R} (4, major *syn*-enantiomer) = 25.67 min).

### (2S,3S)-S-(2-fluorophenyl) 2-fluoro-3-hydroxy-3-(4-((phenylsulfonyl)oxy)phenyl) propanethioate:

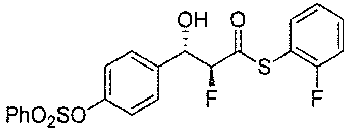

(48% yield, dr 9:1 *synlanti*); **¹H NMR** (400 MHz, Acetone-*d*₆) δ = 5.23 (br d, *J =* 22.8 Hz, 1 H), 5.31-5.43 (m, 1 H), 5.46 (dd, *J* = 48.7, 3.3 Hz, 1H), 7.05-7.10, 7.29-7.35, 7.42-7.44, 7.55-7.68, 7.73-7.82, 7.86-7.91 (6m, 2H, 3H, 2H, 3H, 1H, 2H) ppm. **¹³C NMR** (101 MHz, Acetone-*d*₆) δ = 72.9 (d, *J =* 20.0 Hz), 97.7 (d, *J* = 194.7 Hz), 113.2 (dd, *J* = 18.9, 6.4 Hz), 116.1 (d, *J* = 22.6 Hz), 121.8, 125.1 (d, *J =* 3.7 Hz), 128.4, 128.8, 129.5, 132.9 (d, *J =* 8.2 Hz), 134.6, 135.3, 136.9, 137.4 (d, *J* = 3.7 Hz), 149.4, 162.4 (d, J = 248.8 Hz), 193.4 (d, *J* = 29.5 Hz) ppm. Visible signals of the *syn* diastereoisomer δ = 72.3 (d, *J* = 18.8 Hz), 97.9 (d, *J =* 195.2 Hz), 122.0, 128.3, 129.5, 134.6, 137.0, 149.2 ppm. **¹⁹F NMR** (377 MHz, Acetone-*d*₆) δ = -107.6, -198.4 (ddd, *J =* 49.1, 23.1, 3.9 Hz, *anti*-isomer), -107.7, -204.22 (dd, *J* = 46.7, 27.3 Hz, *syn*-isomer) ppm. **IR** (neat): ṽ = 3525, 3314, 3068, 2920, 2852, 1702, 1670, 1634, 1597, 1503, 1477, 1449, 1374, 1264, 1229, 1200, 1179, 1153, 1092, 1070, 1018, 868, 756, 686 cm⁻¹. **HRMS** (ESI): C₂₁H₁₆F₂O₅S₂ calcd. [M+Na]⁺ = 473.0299, found: 473.0298. **HPLC:** Daicel Chiracel IC, 30% *i*-PrOH/hexane, 0.5 mL/min., 25°C, *anti* 88%ee, *syn* 91 %ee, (t_{R} (1, minor *anti*-enantiomer) = 14.35 min, t_{R} (2, major *anti*-enantiomer) = 15.58 min, t_{R} (3, minor *syn*-enantiomer) = 25.33 min, t_{R} (4, major *syn*-enantiomer) = 29.94 min).

### (2S,3S)-S-(4-methoxyphenyl) 3-(4-bromophenyl)-2-fluoro-3-hydroxypropane-thioate:

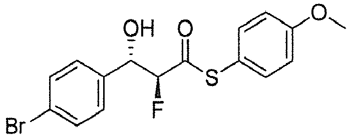

(78% yield, dr 6:1 *anti*/*syn*); **¹H NMR** [400 MHz, C₆D₆/Acetone-*d*₆ (3:1)] δ = 3.48 (s, 3H), 5.00-5.37 (m, 3H), 6.81-6.86, 7.22-7.46 (2m, 2H, 6H) ppm. **¹³C NMR** [101 MHz, C₆D₆/Acetone-*d*₆ (3:1)] δ = 55.0, 73.6 *(d, J =* 21.1 Hz), 97.8 *(d, J =* 195.4 Hz), 115.3, 116.7 *(d, J =* 5.6 Hz), 122.3, 129.8, 131.5, 136.7, 138.0, 161.4, 196.3 (d, *J =* 27.8 Hz) ppm. Visible signals of the *syn* diastereoisomer δ = 73.1 (d, *J =* 18.9 Hz), 98.2 (d, *J =* 197.0 Hz), 117.1 (d, *J =* 5.8 Hz), 122.0, 129.1, 131.6, 136.8, 139.3 ppm. **¹⁹F NMR** [377 MHz, C₆D₆/Acetone-*d*₆-(3:1)] δ = -197.3 (dd, *J* = 48.2, 22.0 Hz, *anti*-isomer), -204.1 (dd, *J* = 46.7, 27.2 Hz, *syn*-isomer) ppm. **IR** (neat): ṽ = 3481, 3007, 2941, 2839, 1691, 1593, 1574, 1494, 1292, 1251, 1175, 1097, 1071, 1030, 1011, 827, 798, 732 cm⁻¹. **HRMS** (ESI): C₁₆H₁₄BrFO₃S calcd. [M+Na]⁺ = 406.9723, found: 406.9720. **HPLC:** Daicel Chiracel OD-H, 30% i-PrOH/hexane, 0.6 mL/min., 25°C, *anti* 96%ee, *syn* 96%ee, (t_{R} (1, minor *anti*-enantiomer) = 11.24 min, t_{R} (2, major *anti*-enantiomer) = 12.99 min, t_{R} (3, minor *syn*-enantiomer) = 19.64 min, t_{R} (4, major *syn*-enantiomer) = 37.14 min).

### (2S,3S)-S-(4-Methoxyphenyl) 3-(4-chlorophenyl)-2-fluoro-3-hydroxypropane-thioate:

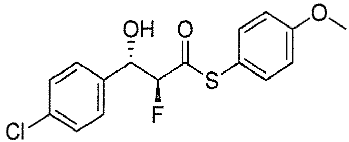

(69% yield, dr 5:1 *anti*/*syn*); anti-isomer: **¹H NMR** [400 MHz, C₆D₆/Acetone-*d*₆ (3:1)] δ = 3.41 (s, 3H), 5.15-5.27 (m, 3H), 6.71-6.74, 7.08-7.12, 7.17-7.21, 7.31-7.33 (4m, 4x2H) ppm. **¹³C NMR** [101 MHz, C₆D₆/Acetone-*d*₆ (3:1)] δ = 55.1, 73.5 (d, *J* = 20.5 Hz), 97.9 (d, *J* = 195.3 Hz), 115.3, 116.7 (d, *J* = 5.7 Hz), 128.4, 129.5, 134.0, 136.7, 137.6 (d, *J* = 3.2 Hz), 161.4, 196.3 (d, *J* = 28.1 Hz) ppm. **¹⁹F NMR** [377 MHz, C₆D₆/Acetone-*d*₆(3:1), ¹H decoupled] δ =-197.6 ppm. **IR** (neat): ṽ = 3475, 3007, 2940, 2839, 1691, 1593, 1574, 1495, 1463, 1440, 1408, 1292, 1251, 1175, 1093, 1030, 1015, 828, 798, 757, 739 cm⁻¹. **HRMS** (ESI): C₁₆H₁₄CIFO₃S calcd. [M+Na]⁺ = 363.0228, found: 363.0230.
*syn*-isomer (2R, 3*S*):¹**H NMR** [400 MHz, C₆D₆/Acetone-*d*₆(3:1)] δ = 3.31 (s, 3H), 4.79 (br s, 1 H), 4.90 (dd, *J* = 47.2, 2.5 Hz, 1 H), 5.22 (ddd, *J* = 26.6, 6.0, 2.5 Hz, 1 H), 6.69-6.73, 7.12-7.15, 7.21-7.28 (3m, 2x2H, 4H) ppm. ¹⁹**F NMR** [377 MHz, C₆D₆/Acetone-*d*₆ (3:1), ¹H decoupled] δ = -203.9 ppm. **IR** (neat): ṽ = 3402, 3016, 2959, 2926, 2846, 1687, 1593, 1572, 1495, 1459, 1439, 1406, 1321, 1291, 1252, 1174, 1090, 1027, 1013, 990, 896, 851, 827, 765, 738 cm⁻¹. **HRMS** (ESI): C₁₆H₁₄CIFO₃S calcd. [M+Na]⁺ = 363.0228, found: 363.0228. **HPLC:** Daicel Chiracel OD-H, 30% i-PrOH/hexane, 0.6 mL/min., 25°C, *anti* 96%ee, *syn* 96%ee, (t_{R} (1, minor *anti*-enantiomer) = 10.78 min, t_{R} (2, major *anti*-enantiomer) = 12.32 min, t_{R} (3, minor *syn*-enantiomer) = 18.24 min, t_{R} (4, major *syn*-enantiomer) = 33.94 min).

### (2S,3S)-S-(4-Methoxyphenyl) 3-(3-cyanophenyl)-2-fluoro-3-hydroxy propanethioate:

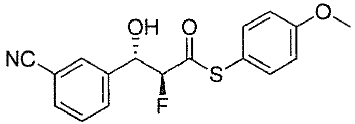

(99% yield, dr 5:1 *anti*/*syn*); **¹H NMR** (400 MHz, C₆D₆) δ = 3.07 (br s, 1 H), 3.31 (s, 3H), 4.64-5.03 (m, 2H), 6.75-6.82, 6.86-6.94, 7.07-7.13, 7.21-7.33, 7.38-7.49 (5m, 2H, 2×1H, 2×2H) ppm. **¹³C NMR** (101 MHz, C₆D₆) δ = 54.9, 73.2 (d, *J =* 20.7 Hz), 96.8 (d, *J =* 195.4 Hz), 112.7, 115.5, 116.1 (d, *J=* 5.6 Hz), 118.9, 129.1, 131.0, 313.7, 132.0, 136.6, 139.3, 161.53, 197.1 (d, *J* = 27.6 Hz) ppm. Visible signals of the *syn* diastereoisomer δ = 72.8 (d, *J* = 19.0 Hz), 97.2 (d, *J* = 198.5 Hz), 112.8, 116.5 (d, *J* = 5.7 Hz), 129.2, 130.3, 131.8, 136.7, 140.4, 161.47 ppm. **¹⁹F NMR** (377 MHz, C₆D₆) δ = -196.0 (dd, *J* = 49.2, 17.8 Hz, *anti*-isomer), -203.7 (dd, *J* = 47.3, 25.9 Hz, *syn*-isomer) ppm. **IR** (neat): ṽ = 3463, 3073, 3010, 2929, 2840, 2232, 1693, 1593, 1574, 1495, 1463, 1439, 1408, 1292, 1251, 1175, 1096, 1080, 1029, 829, 809, 762, 740, 700 cm⁻¹. **HRMS** (ESI): C₁₇H₁₄FNO₃S calcd. [M+Na]⁺ = 354.0571, found: 354.0569. **HPLC:** Daicel Chiracel OD-H, 30% *i*-PrOH/hexane, 0.6 mL/min., 25°C, *anti* 95%ee, *syn* 94%ee, (t_{R} (1, minor *anti*-enantiomer) = 12.74 min, t_{R} (2, major *anti*-enantiomer) = 14.41 min, t_{R} (3, minor *syn*-enantiomer) = 59.79 min, t_{R} (4, major *syn*-enantiomer) = 88.48 min).

### (2S,3S)-S-(4-Methoxyphenyl)-2-fluoro-3-hydroxy-3-(2-nitrophenyl)propane-thioate:

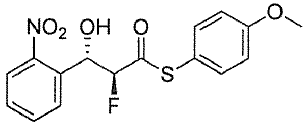

(61% yield, dr 3:1 ***anti*/*syn*); ¹H NMR** (300 MHz, CDCl₃) δ = 7.95-7.99, 7.85-7.75, 7.67-7.55, 7.47-7.39, 7.33-7.19, 6.96-6.78 (6m, 4×1H, 2×2H), 5.85 (dd, *J=* 11.4, 4.7 Hz, 1H), 5.17 (dd, *J* = 47.0, 4.7 Hz, 1H), 3.75 (s, 3H), 2.80 (br s, 1 H) ppm. Visible signals of the minor diastereoisomer δ = 5.92 (d, *J =* 2.4 Hz), 5.32 (dd, *J* = 50.0, 3.0 Hz), 3.76 (s) ppm. **¹³C NMR** (some additional signals of the minor diastereoisomer visible in the aromatic region) (75 MHz, CDCl₃) δ = 197.45 (d, *J* = 29.0 Hz), 161.2, 148.0, 136.5, 136.4, 133.9, 133.8, 132.7, 132.6, 130.2, 129.67, 129.65, 129.42, 129.38, 124.9, 124.85, 115.9, 115.8, 115.3, 96.14 (d, *J =* 192.5 Hz), 69.7 (d, *J* = 23.7 Hz), 55.5 ppm. **¹⁹F NMR** (282 MHz, CDCl₃) δ = -188.9 (major diastereoisomer), -205.1 (minor diastereoisomer) ppm. IR (neat): ṽ = 3491, 3008, 2941, 2840, 1692, 1592, 1575, 1523, 1495, 1342, 1292, 1248, 1175, 1085, 1064, 1027, 911, 828, 789, 733 cm⁻¹. **HRMS** (ESI): C₁₆H₁₄FNO₅S calcd. [M+Na]⁺ = 374.0469, found: 374.0467. HPLC: Daicel Chiracel OD-H, 30% *i*-PrOH/hexane, 0.6 mL/min., 25°C, *anti* 96%ee, *syn* 96%ee, (t_{R} (1, minor *anti*-enantiomer) = 12.52 min, t_{R} (2, minor *syn*-enantiomer) = 13.74 min, t_{R} (3, major *anti*-enantiomer) = 15.02 min, t_{R} (4, major *syn*-enantiomer) = 17.26 min).

### (2S,3S)-S-(4-Methoxyphenyl) 3-(benzofuran-2-yl)-2-fluoro-3-hydroxypropane-thioate:

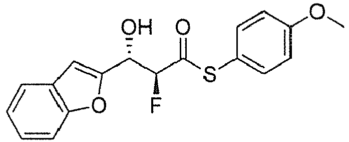

(87% yield, dr 3:1 *anti*/*syn*); **¹H NMR** [400 MHz, C₆D₆/Acetone-*d*₆ (3:1)] δ = 3.41 (s, 3H), 5.24-5.62 (m, 3H), 6.77-6.97, 7.14-7.21, 7.28-7.50 (3m, 3H, 2H, 4H) ppm. **¹³C NMR** [101 MHz, C₆D₆/Acetone-*d*₆ (3:1)] δ = 55.0, 69.3 (d, *J* = 22.0 Hz), 96.6 (d, *J* = 195.7 Hz), 105.7, 111.5, 115.27, 116.8 (d, J = 5.4 Hz), 121.6, 123.2, 124.7, 128.6, 136.7, 154.9 (d*, J* = 4.2 Hz), 155.39, 161.3, 195.4 (d, *J* = 28.0 Hz) ppm. Visible signals of the *syn* diastereoisomer δ = 69.0 (d, *J=* 19.3 Hz), 96.2 (d, *J =* 197.3 Hz), 115.31, 116.9 (d, *J* = 5.7 Hz), 121.5, 123.3, 128.7, 136.8, 155.35, 155.8 (d, *J* = 2.3 Hz), 161.4, 196.4 (d, *J* = 29.3 Hz) ppm. **¹⁹F NMR** [377 MHz, C₆D₆/Acetone-*d*₆(3:1)] δ = -196.8 (dd, *J* = 47.8, 20.9 Hz, *anti*-isomer), -203.6 (dd, *J =* 47.3, 26.4 Hz, *syn*-isomer) ppm. **IR** (neat): ṽ = 3460, 3116, 3065, 3007, 2941, 2838, 2280, 1774, 1691, 1592, 1494, 1453, 1407, 1291, 1248, 1174, 1106, 1080, 1027, 965, 826, 811, 750 cm⁻¹. **HRMS** (ESI): C18H15FO4S calcd. [M+Na]⁺ = 369.0567, found: 369.0569. **HPLC:** Daicel Chiracel OD-H, 30% *i*-PrOH/hexane, 0.6 mL/min., 25°C, *anti* 79%ee, *syn* 80%ee, (t_{R} (1, minor *anti*-enantiomer) = 13.58 min, t_{R} (2, major *anti*-enantiomer) = 14.30 min, t_{R} (3, minor *syn*-enantiomer) = 17.24 min, t_{R} (4, major *syn*-enantiomer) = 21.85 min).

### (2S,3S)-S-(2-Fluorophenyl) 2-fluoro-3-hydroxy-5-(methylthio)pentanethioate:

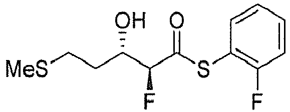

(41% yield, dr 9:1 *anti*/*syn*); **¹H NMR** [400 MHz, C₆D₆/Acetone-*d*₆ (3:1)] δ = 1.78 (s, 3H), 1.85 (dtd, *J =* 14.1, 8.7, 2.9 Hz, 1H), 1.99-2.08, 2.45-2.60 (2m, 2×2H), 3.96 (d, J = 6.0 Hz, 1 H), 4.26 (dddt, *J* = 22.1, 8.7, 6.0, 3.1 Hz, 1 H), 4.88 (dd, *J* = 48.7, 3.1 Hz, 1H), 6.73-6.77, 6.80-6.86, 6.91-6.97, 7.17-7.20 (4m, 4×1H) ppm. Visible signals of the *syn* diastereoisomer δ = 3.78 (d, *J* = 7.7 Hz, 1 H), 4.14-4.21 (m, 1H), 4.64 (dd, *J =* 47.9, 2.6 Hz, 1H) ppm. **¹³C NMR** [101 MHz, C₆D₆/Acetone-*d*₆(3:1)] δ = 14.8, 30.3, 30.5 (d, *J* = 5.1 Hz), 70.8 (d, *J* = 20.3 Hz), 98.6 (d, *J* = 192.3 Hz), 114.1 (dd, *J =* 18.8, 6.7 Hz), 116.4 (d, *J* = 22.7 Hz), 125.0 (d, *J* = 3.8 Hz), 132.6 (d, *J* = 8.1 Hz), 137.1, 163.0 (d, *J* = 249.4 Hz), 194.4 (d, *J* = 30.3 Hz) ppm. Visible signals of the *syn* diastereoisomer δ = 15.0, 32.2 (d, *J* = 3.6 Hz), 70.4 (d, *J* = 19.4 Hz), 98.1 (d, *J* = 192.9 Hz), 124.9 (d, *J* = 3.9 Hz), 132.5 (d, *J* = 8.3 Hz), 137.3 ppm. **¹⁹F NMR** [377 MHz, C₆D₆/Acetone-*d*₆ (3:1), ¹H-decoupled] δ = -106.5, -196.9 (*anti*-isomer), -106.4, -203.3 (*syn*-isomer) ppm. **IR** (neat): ṽ = 3405, 3074, 2920, 1703, 1596, 1581, 1477, 1448, 1327, 1264, 1228, 1105, 1068, 974, 827, 758, 699 cm⁻¹. **HRMS** (ESI): C₁₂H₁₄F₂O₂S₂ calcd. [M+Na]⁺ = 315.0295, found: 315.0298. **HPLC:** Daicel Chiracel OD-H, 2% EtOH/hexane, 1.2 mL/min., 8°C, *anti* 71%ee, *syn* 65%ee, (t_{R} (1, minor *anti-*enantiomer) = 27.67 min, t_{R} (2, major *anti*-enantiomer) = 28.97 min, t_{R} (3, minor *syn-*enantiomer) = 55.25 min, t_{R} (4, major *syn*-enantiomer) = 87.38 min).

### (2S,3S)-S-(2-Fluorophenyl) 2-fluoro-3-hydroxy-4-phenylbutanethioate:

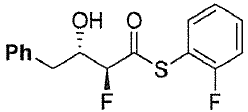

(46% yield, dr 10:1 *anti*/*syn*); **¹H NMR** [400 MHz, C₆D₆/Acetone-*d*₆ (3:1)] δ = 2.91-3.03, 4.28-4.37 (2m, 2×2H), 4.95 (dd, *J* = 48.2, 2.8 Hz, 1H), 6.82-6.92, 7.00-7.10, 7.16-7.24 (3m, 2H, 4H, 3H) ppm. Visible signals of the *syn* diastereoisomer δ = 4.73 (dd, *J* = 47.5, 2.1 Hz, 1H) ppm. **¹³C NMR** [101 MHz, C₆D₆/Acetone-*d*₆(3:1)] δ = 38.1 (d, *J* = 6.3 Hz), 73.6 (d, *J* = 20.3 Hz), 98.0 (d, *J* = 191.8 Hz), 114.2 (dd, *J* = 18.8, 6.9 Hz), 116.4 (d, *J =* 22.7 Hz), 125.1 (d, *J* = 3.8 Hz), 126.7, 128.6, 130.0, 132.7 (d, *J =* 8.2 Hz), 137.3, 138.3, 162.9 (d, *J* = 249.2 Hz), 194.5 (d, *J* = 30.5 Hz) ppm. Visible signals of the *syn* diastereoisomer δ = 39.6, 73.0 (d, *J* = 19.1 Hz), 97.0 (d, *J* = 193.5 Hz), 116.35 (d, *J=* 22.6 Hz), 125.0 (d, *J =* 3.9 Hz), 126.8, 128.8, 129.7, 132.5 (d, *J=* 8.2 Hz), 137.4, 137.9 ppm. **¹⁹F NMR** [377 MHz, C₆D₆/Acetone-*d*₆ (3:1)] δ = -106.8, -196.5 (*anti*-isomer), -106.8, -206.5 (*syn*-isomer) ppm. **IR** (neat): ṽ = 3440, 3064, 3030, 2925, 1702, 1597, 1580, 1496, 1476, 1448, 1366, 1264, 1228, 1105, 1069, 1028, 942, 826, 757, 700 cm⁻¹. **HRMS** (ESI): C₁₆H₁₄F₂O₂S calcd. [M+Na]⁺ = 331.0575, found: 331.0579. **HPLC:** Daicel Chiracel OD-H, 4% EtOH/hexane, 1.2 mL/min., 8°C, *anti* 71%ee, *syn* 69%ee, (t_{R} (1, minor *anti*-enantiomer) = 14.27 min, t_{R} (2, minor *syn-*enantiomer) = 19.21 min, t_{R} (3, major *anti*-enantiomer) = 20.68 min, t_{R} (4, major *syn-*enantiomer) = 27.47 min).

### (2S,3S)-S-(2-Fluorophenyl)-2-fluoro-3-hydroxy-5-phenylpentanethioate:

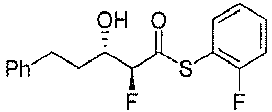

(48% yield, dr 9:1 *anti*/*syn*); **¹H NMR** [400 MHz, C₆D₆/Acetone-*d*₆(3:1)] δ = 1.97 (dddd, *J* = 14.1, 10.0, 7.1, 3.3 Hz, 1 H), 2.13 (dtd, *J* = 14.1, 9.6, 4.8 Hz, 1 H), 2.68 (ddd, *J* = 14.0, 9.6, 7.1 Hz, 1 H), 2.91 (ddd, *J =* 14.0, 10.0, 4.8 Hz, 1 H), 3.85 (d, *J* = 6.2 Hz, 1 H), 4.09 (dddt, *J* = 21.1, 9.6, 6.2, 3.3 Hz, 1H), 4.88 (dd, *J* = 48.6, 3.3 Hz, 1 H), 7.24-7.14, 7.12-7.06, 7.02-6.95, 6.91-6.85, 6.81-6.77 (5m, 5H, 4×1H). Visible signals of the minor diastereoisomer δ = 1.79 (dddd, *J =* 14.0, 10.0, 6.9, 4.2 Hz, 1H), 2.62-2.55 (m, 1H), 2.80 (ddd, *J* = 14.6, 10.0, 5.1 Hz, 1H), 3.66 (d, *J* = 8.0 Hz, 1H), 4.67 (dd, *J* = 47.9, 2.6 Hz, 1 H) ppm. **¹³C NMR** [101 MHz, C₆D₆/Acetone-*d*₆(3:1)] δ = 32.04, 33.16 (d, *J =* 5.1 Hz), 71.47 (d, *J* = 20.4 Hz), 98.50 (d, *J* = 191.9 Hz), 116.38 (d, *J* = 22.6 Hz), 124.96 (d, *J* = 3.9 Hz), 126.18, 128.77 (d, J = 13.2 Hz), 132.54 (d, *J* = 8.2 Hz), 137.15, 141.96, 161.70, 164.18, 194.45 (d, *J* = 30.3 Hz) ppm. ¹⁹**F NMR** [377 MHz, C₆D₆/Acetone-*d*₆ (3:1)] δ = -106.48, -196.47 (major diastereoisomer), -203.85 (minor diastereoisomer) ppm. **IR** (neat): ṽ = 3433, 3064, 3028, 2926, 2861, 1702, 1597, 1581, 1496, 1476, 1449, 1263, 1228, 1107, 1060, 1028, 944, 826, 756, 699 cm⁻¹. **HRMS** (ESI): C₁₇H₁₆F₂O₂S calcd. [M+Na]⁺ = 345.0731, found: 345.0737. HPLC: Daicel Chiracel OD-H, 10% *i*-PrOH/hexane, 0.7 mL/min., 25°C, *anti* 44%ee, *syn* 46%ee, (t_{R} (1, minor *anti-*enantiomer) = 10.00 min, t_{R} (2, major *anti*-enantiomer) = 11.07 min, t_{R} (3, minor *syn-*enantiomer) = 16.38 min, t_{R} (4, major *syn*-enantiomer) = 21.47 min).

### (2S,3S)-S-(2-Fluorophenyl) 2-fluoro-3-hydroxy-3-(thiophen-3-yl)propanethioate:

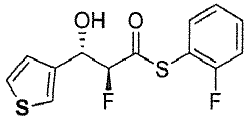

(43% yield, dr 4:1 *anti*/*syn*); **¹H NMR** [400 MHz, C₆D₆/Acetone-*d*₆(3:1)] δ = 4.81 (d, *J* = 5.3 Hz, 1 H), 5.19 (dd, *J* = 49.0, 3.5 Hz, 1H), 5.23-5.31 (m, 1H), 6.76-6.86, 6.95-7.02, 7.07-7.11, 7.16-7.21 (4m, 2×2H, 1H, 2H) ppm.
Visible signals of the minor diastereoisomer δ = 4.72 (d, *J* = 6.9 Hz, 1 H), 4.95 (dd, *J =* 47.2, 2.4 Hz, 1 H). **¹³C NMR** [101 MHz, C₆D₆/Acetone-*d*₆ (3:1)] δ = 70.6 (d, *J* = 20.5 Hz), 97.7 (d, *J =* 195.5 Hz), 113.6 (dd, *J=* 18.8, 6.4 Hz), 116.0 (d, *J =* 22.5 Hz), 123.1, 124.6 (d, *J =* 3.8 Hz), 125.3, 126.9 (d, *J=* 1.4 Hz), 132.2 (d, *J =* 8.1 Hz), 136.7, 139.2 (d, *J* = 3.4 Hz), 162.5 (d, *J* = 249.7 Hz), 193.5 (d, *J* = 29.3 Hz) ppm. ¹⁹**F NMR** [377 MHz, C₆D₆/Acetone-*d*₆ (3:1)] δ = -106.5, -197.4 (dd, *J* = 49.0, 23.6 Hz). (major diastereoisomer), -106.6, -202.5 (dd, *J* = 47.1, 27.1 Hz) (minor diastereoisomer) ppm. **IR** (neat): ṽ = 3449, 3111, 2926, 2858, 1702, 1596, 1581, 1477, 1449, 1264, 1229, 1108, 1083, 1069, 858, 828, 806, 758, 667 cm⁻¹. **HRMS** (ESI): C₁₃H₁₀F₂O₂S₂ calcd. [M+Na]⁺ = 322.9982, found: 322,9989. HPLC: Daicel Chiracel IC, 10% i-PrOH/hexane, 0.7 mL/min., 25°C, *anti* 82%ee, *syn* 88%ee, (t_{R} (1, minor *anti*-enantiomer) =11.88 min, t_{R} (2, major *anti*-enantiomer) = 14.28 min, t_{R} (3, minor *syn*-enantiomer) = 17.28 min, t_{R} (4, major *syn*-enantiomer) = 22.88 min).

### (2S,3S)-S-(2-Fluorophenyl) 4-(benzyloxy)-2-fluoro-3-hydroxybutanethioate:

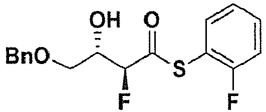

(73% yield, dr 10:1 *anti*/*syn*); **¹H NMR** [400 MHz, C₆D₆/Acetone-*d*₆ (3:1)] δ = 3.59-3.73, 4.27-4.34, 4.40-4.43 (3m, 2H, 3H, 1H), 5.12 (dd, *J* = 48.1, 2.5 Hz, 1H), 6.75-6.89, 6.97-7.03, 7.05-7.20 (3m, 2H, 1 H, 6H) ppm. Visible signals of the minor diastereoisomer δ = 5.10 (dd, *J=* 47.4,1.8 Hz, 3H). **¹³C NMR** [101 MHz, C₆D₆/Acetone-*d*₆ (3:1)] δ = 69.2 (d, *J* = 7.5 Hz), 71.0 (d, *J* = 20.4 Hz), 73.0, 96.4 (d, *J =* 191.8 Hz), 113.8 (dd, *J* = 18.7, 6.6 Hz), 115.9 (d, *J =* 22.6 Hz), 124.6 (d, *J =* 3.8 Hz), 127.3, 127.5, 128.1, 132.1 (d, *J* = 8.2 Hz), 137.0, 138.2, 162.5 (d, *J* = 249.2 Hz), 193.4 (d, *J* = 30.3 Hz) ppm. Visible signals of the minor diastereoisomer δ = 69.4 (d, *J* = 4.8 Hz), 70.9 (d, *J =* 20.3 Hz), 72.9, 95.4 *(*d, *J* = 193.2 Hz), 115.9 (d, *J* = 22.6 Hz), 132.1 (d, *J* = 8.1 Hz), 136.9, 138.2, 194.8 (d, *J =* 31.1 Hz). **¹⁹F NMR** [377 MHz, C₆D₆/Acetone-*d*₆(3:1)] δ =-106.8 (dt, *J* = 9.5, 6.6 Hz), -199.3 (dd, *J* = 48.1, 21.2 Hz) (major diastereoisomer), - 106.7 (dt, *J* = 8.8, 6.7 Hz), -208.0 (dd, *J* = 47.4, 27.3 Hz), (minor diastereoisomer) ppm. **IR** (neat): ṽ = 3438, 3064, 3030, 2923, 2868, 1703, 1579, 1476, 1449, 1365, 1263, 1228, 1093, 1070, 1027, 826, 736, 698 cm⁻¹. **HRMS** (ESI): C₁₇H₁₆F₂O₃S calcd. [M+Na]⁺ = 361.0680, found: 361.0685. **HPLC:** Daicel Chiracel IC, 10% *i*-PrOH/hexane, 0.7 mL/min., 25°C, *anti* 80%ee, *syn* 77%ee, (t_{R} (1, major *anti*-enantiomer) = 17.81 min, t_{R} (2, minor *anti*-enantiomer) = 20.48 min, t_{R} (3, minor *syn*-enantiomer) = 25.86 min, t_{R} (4, major *syn*-enantiomer) = 30.92 min).

### (2S,3S)-S-(2-Fluorophenyl) 5-(1,3-dioxoisoindolin-2-yl)-2-fluoro-3-hydroxy-pentanethioate:

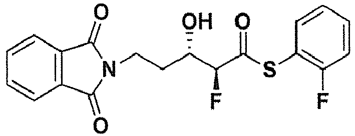

(82% yield, dr 7:1 *anti*/*syn*); **¹H NMR** [400 MHz, C₆D₆/Acetone-*d*₆(3:1)] δ = 1.97 (dtd, *J =* 14.2, 7.6, 2.9 Hz, 1H), 2.08 (dddd, *J* = 14.2, 10.3, 6.8, 5.5 Hz, 1H), 2.27-2.33, 3.65-3.81,4.05-4.15, (3m, 1H, 2H, 1 H), 4.86 (dt, *J =* 48.6, 2.9 Hz, 1H), 6.74-6.85, 6.92-6.97, 7.07-7.11, 7.18-7.22, 7.44-7.49 (5m, 2H, 1 H, 2H, 1 H, 2H) ppm. Visible signals of the minor diastereoisomer δ = 4.68 (dt, *J =* 49.3, 2.5 Hz, 1 H). **¹³C NMR** [101 MHz, C₆D₆/Acetone-*d*₆ (3:1)] δ = 30.0 (d, *J* = 5.6 Hz), 34.5, 69.7 (d, *J* = 20.6 Hz), 97.8 (d, *J* = 192.4 Hz), 113.6 (dd, *J* = 18.8, 6.8 Hz), 115.9 (d, *J =* 22.6 Hz), 122.7, 124.6 (d, *J =* 3.8 Hz), 132.1, 132.2 (d, *J* = 8.0 Hz), 133.3, 136.8, 162.5 (d, *J =* 249.5 Hz), 167.9, 193.9 (d, *J =* 30.4 Hz) ppm. Visible signals of the minor diastereoisomer δ = 34.4, 69.0 (d, *J =* 19.2 Hz), 97.5 (d, *J =* 193.4 Hz), 133.4, 136.9. **¹⁹F NMR** [377 MHz, C₆D₆/Acetone-*d*₆(3:1)] δ = -106.5, -196.2 (dd, *J* = 48.6, 21.4 Hz). (major diastereoisomer), -204.5 (dd, *J* = 49.3, 26.2 Hz) (minor diastereoisomer) ppm. **IR** (neat): ṽ = 3467, 3068, 2949, 1771, 1704, 1615, 1596, 1581, 1477, 1440, 1398, 1375, 1264, 1229, 1128, 1089, 1071, 961, 827, 761, 721 cm⁻¹. **HRMS** (ESI): Chemical Formula: C₁₉H₁₅F₂NO₄S calcd. [M+Na]⁺ = 414.0582, found: 414.0584. HPLC: Daicel Chiracel OD-H, 45% *i*-PrOH/hexane, 0.5 mL/min., 8°C, *anti* 85%ee, *syn* 86%ee, (t_{R} (1, major *anti*-enantiomer) = 22.82 min, t_{R} (2, minor *anti-*enantiomer) = 25.54 min, t_{R} (3, minor *syn*-enantiomer) = 30.95 min, t_{R} (4, major *syn-*enantiomer) = 62.17 min).

### (2R,3R)-S-(2-fluorophenyl) 2-fluoro-5-(2-(4-fluorophenyl)-5-isopropyl-3-phenyl-4-(phenylcarbamoyl)-1H-pyrrol-1-yl)-3-hydroxypentanethioate:

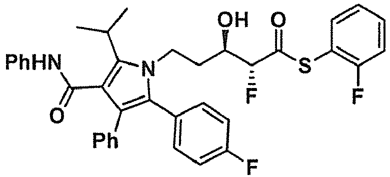

(97% yield, dr 5:1 *anti*/*syn*); **¹H NMR** (400 MHz, Acetone-*d*₆) δ = 1.47 (d, *J=* 7.1 Hz, 6H), 1.78-2.10 (m, 2H), 3.41 (vis p, *J =* 7.1 Hz, 1 H), 3.92-4.09 (m, 2H), 4.21 (ddd, *J =* 14.7, 11.0, 5.0 Hz, 1 H), 4.84 (d, *J* = 6.0 Hz, 1 H), 5.20 (dd, *J* = 48.4, 2.8 Hz, 1 H), 6.95-7.66 (m, 18H), 8.39 (br s, 1 H) ppm. Visible signals of the minor diastereoisomer δ = 4.67 (d, *J* = 7.5 Hz, 1 H), 5.09 (dd, *J* = 47.5, 2.8 Hz, 1 H). **¹³C NMR** [101 MHz, CDCl₃/DMSO-*d*₆ (6:1)] δ = 21.2, 25.5, 32.0, 40.9, 68.7 (d, *J* = 19.9 Hz), 97.4 (d, *J =* 193.7 Hz), 112.5 (dd, *J=* 18.7, 6.8 Hz), 114.8 (d, *J =* 21.6 Hz), 115.2, 115.7 *(d, J =* 22.6 Hz), 119.1, 120.6, 121.1, 122.9, 124.4 (d, *J* = 3.5 Hz), 125.7, 126.0 (d, *J* = 4.1 Hz), 127.6, 128.0, 129.6, 132.1 (d, *J* = 7.7 Hz), 132.6 (d, *J* = 8.2 Hz), 134.1, 136.0, 138.0, 139.8, 161.6 (d, *J* = 245.8 Hz), 161.6 (d, *J* = 249.0 Hz), 164.6, 193.5 (d, *J* = 28.9 Hz) ppm. **¹⁹F NMR** (377 MHz, Acetone-*d*₆) δ = -107.7, -115.5 -199.0 (dd, *J =* 48.3, 23.8 Hz). (major diastereoisomer), -204.2 (dd, *J* = 47.6, 26.6 Hz), (minor diastereoisomer) ppm. IR (neat): ṽ = 3423, 3336, 3056, 2970, 2934, 2874, 1700, 1664, 1626, 1597, 1558, 1523, 1508, 1478, 1438, 1365, 1313, 1225, 1158, 1113, 1070, 845, 807, 759, 725, 704, 692 cm⁻¹. **HRMS** (ESI): Chemical Formula: Chemical Formula: C₃₇H₃₃F₃N₂O₃S calcd. [M+Na]⁺ = 665.2056, found: Exact Mass: 665.2059. HPLC: Daicel Chiracel IC, 50% CH₂Cl₂/hexane, 1.0 mL/min., 45°C, *anti* 50%ee, *syn* 60%ee, (t_{R} (1, minor *anti*-enantiomer) = 15.43 min, t_{R} (2, major *anti*-enantiomer) = 18.01 min, t_{R} (3, minor *syn*-enantiomer) = 23.59 min, t_{R} (4, major *syn*-enantiomer) = 25.78 min).

## Claims

1. A compound of formula (I) 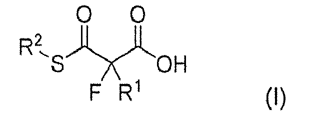 wherein
R¹ represents hydrogen, halogen, an optionally substituted alkyl group, an optionally substituted cycloalkyl group, an optionally substituted aryl group; and
R² represents an optionally substituted aryl group, an optionally substituted cycloalkyl group, an optionally substituted heteroaryl group, an optionally substituted heterocyclyl group; an optionally substituted alkyl group; and
wherein R² does not represent the CoA-residue.

2. The compound of formula (I), wherein
R¹ represents hydrogen, halogen, a (C₁-C₈)alkyl group, a (C₃-C₈)cycloalkyl or an aryl group; wherein said (C₁₋₈)alkyl group is unsubstituted or mono-, di-, tri or tetra-substituted, the optional substituent(s) on the said (C₁₋₈)alkyl moiety being independently selected from the group consisting of halogen, cyano, oxo, nitro, amino, (C₂₋₄)alkenyl, (C₂₋₄)alkynyl, (C₁₋₈)alkoxy, (C₁₋₈)alkoxy(C₁₋₈)-alkoxy, (C₁₋₈)alkylthio, (C₁₋₈)alkylsulfinyl, (C₁₋₈) alkylsulfonyl, (C₁₋₈) alkylcarbonyloxy, (C₁₋₈)alkoxycarbonyl and (C₁₋₈)alkoxy carbonyloxy, (C₃₋₈) cycloalkyl, (C₃₋₈)cycloalkyl(C₁₋₈)alkyl, (C₃₋₈)cycloalkoxy, (C₃₋₈) cycloalkoxy(C₁₋₈)alkyl, (C₃₋₈)cycloalkyl(C₁₋₈)alkoxy, (C₃₋₈)cycloalkoxy(C₁₋₈) alkoxy, aryl, aryl(C₁₋₈)alkyl, aryloxy, aryloxy(C₁₋₈)alkyl, aryl(C₁₋₈)alkoxy, aryloxy(C₁₋₈)alkoxy, carboxy, carbamyl, hydroxy, (C₁₋₈)alkoxy, (C₁₋₈) alkoxy(C₁₋₈)alkoxy, (C₁₋₈)alkoxy substituted by halogen, (C₁₋₈)alkoxy(C₁₋₈) alkyl, (C₁₋₈)alkylthio, (C₁₋₈)alkylthio(C₁₋₈)alkyl, (C₁₋₈)alkylsulfinyl, (C₁₋₈) alkylsulfinyl(C₁₋₈)alkyl, (C₁₋₈)alkylsulfonyl, (C₁₋₈)alkylsulfonyl(C₁₋₈)alkyl, (C₁₋₈) alkylamino, di(C₁₋₈)alkylamino with two identical or different (C₁₋₈)alkyl moieties, amino(C₁₋₈)alkyl, (C₁₋₈)alkylamino(C₁₋₈)alkyl, di(C₁₋₈) alkylamino(C₁₋₈)alkyl with two identical or different (C₁₋₈)alkyl moieties in the di(C₁₋₈)alkylamino moiety, amino(C₁₋₈)alkoxy, (C₁₋₈)alkylamino(C₁₋₈) alkoxy, di(C₁₋₈)alkylamino(C₁₋₈)alkoxy with two identical or different (C₁₋₈) alkyl moieties, formyl, (C₁₋₈)alkylcarbonyl, formyloxy, (C₁₋₈) alkylcarbonyloxy, formyl(C₁₋₈)alkyl, (C₁₋₈)alkylcarbonyl(C₁₋₈)alkyl, formyl(C₁₋₈)alkoxy, (C₁₋₈)alkylcarbonyl(C₁₋₈)alkoxy, (C₁₋₈)alkoxycarbonyl, (C₁₋₈)alkoxycarbonyloxy, (C₁₋₈)alkoxycarbonyl(C₁₋₈)alkyl, (C₁₋₈)alkoxycarbonyl(C₁₋₈)alkoxyand
wherein said aryl group, (C₃-C₈)cycloalkyl group is unsubstituted, mono-substituted, di-substituted or tetra- substituted, the optional substituent(s) being independently selected from the group consisting of halogen, cyano, nitro, carboxy, carbamyl, hydroxy, (C₁₋₈)alkyl, (C₁₋₈)alkyl substituted by halogen, (C₃₋₈)cycloalkyl, (C₃₋₈)cycloalkyl(C₁₋₈)alkyl, (C₃₋₈)cycloalkoxy, (C₃₋₈) cycloalkoxy(C₁₋₈)alkyl, (C₃₋₈)cycloalkyl(C₁₋₈)alkoxy, (C₃₋₈)cycloalkoxy(C₁₋₈) alkoxy, aryl, aryl(C₁₋₈)alkyl, aryloxy, aryloxy(C₁₋₈)alkyl, aryl(C₁₋₈)alkoxy, aryloxy(C₁₋₈)alkoxy, (C₁₋₈)alkoxy, (C₁₋₈)alkoxy(C₁₋₈)alkoxy, (C₁₋₈)alkoxy substituted by halogen, (C₁₋₈)alkoxy(C₁₋₈)alkyl, (C₁₋₈)alkylthio, (C₁₋₈) alkylthio(C₁₋₈)alkyl, (C₁₋₈)alkylsulfinyl, (C₁₋₈)alkylsulfinyl(C₁₋₈)alkyl, (C₁₋₈) alkylsulfonyl, (C₁₋₈)alkylsulfonyl(C₁₋₈)alkyl, amino, (C₁₋₈)alkylamino, di(C₁₋₈) alkylamino with two identical or different (C₁₋₈)alkyl moieties, amino(C₁₋₈) alkyl, (C₁₋₈)alkylamino(C₁₋₈)alkyl, di(C₁₋₈)alkylamino(C₁₋₈)alkyl with two identical or different (C₁₋₈)alkyl moieties in the di(C₁₋₈)alkylamino moiety, amino(C₁₋₈)alkoxy, (C₁₋₈)alkylamino(C₁₋₈)alkoxy, di(C₁₋₈)alkylamino(C₁₋₈) alkoxy with two identical or different (C₁₋₈)alkyl moieties, formyl, (C₁₋₈)-alkylcarbonyl, formyloxy, (C₁₋₈)alkylcarbonyloxy, formyl(C₁₋₈)alkyl, (C₁₋₈) alkylcarbonyl(C₁₋₈)alkyl, formyl(C₁₋₈)alkoxy, (C₁₋₈)alkylcarbonyl(C₁₋₈)alkoxy, (C₁₋₈)alkoxycarbonyl, (C₁₋₈)alkoxycarbonyloxy, (C₁₋₈)alkoxycarbonyl(C₁₋₈) alkyl, (C₁₋₈)alkoxycarbonyl(C₁₋₈)alkoxy, -OCH₂O- -C(=O)OCH₂-,-CH₂OC(=O)- and -CH=CHCH=CH-, the four last-mentioned optional substituents in each case being attached to two adjacent ring carbon atoms of the said moiety; and
R² represents an aryl group or a (C₃-C₈)cycloalkyl group or a heterocyclyl group with 3 to 10 ring atoms or a heteroaryl group with 3 to 10 ring atoms or a (C₁-C₈)alkyl group;
wherein said aryl group, cycloalkyl group, heterocyclyl group, heteroaryl group, is unsubstituted, mono-substituted, di-substituted or tetra-substituted, the optional substituent(s) being independently selected from the group consisting of halogen, cyano, nitro, carboxy, carbamyl, hydroxy, (C₁₋₈)alkyl, (C₁₋₈)alkyl substituted by halogen, (C₃₋₈)cycloalkyl, (C₃₋₈) cycloalkyl(C₁₋₈)alkyl, (C₃₋₈)cycloalkoxy, (C₃₋₈)cycloalkoxy(C₁₋₈)alkyl, (C₃₋₈) cycloalkyl(C₁₋₈)alkoxy, (C₃₋₈)cycloalkoxy(C₁₋₈)alkoxy, aryl, aryl(C₁₋₈)alkyl, aryloxy, aryloxy(C₁₋₈)alkyl, aryl(C₁₋₈)alkoxy, aryloxy(C₁₋₈)alkoxy, (C₁₋₈) alkoxy, (C₁₋₈)alkoxy(C₁₋₈)alkoxy, (C₁₋₈)alkoxy substituted by halogen, (C₁₋₈) alkoxy(C₁₋₈)alkyl, (C₁₋₈)alkylthio, (C₁₋₈)alkylthio(C₁₋₈)alkyl, (C₁₋₈)alkylsulfinyl, (C₁₋₈)alkylsulfinyl(C₁₋₈)alkyl, (C₁₋₈)alkylsulfonyl, (C₁₋₈)alkylsulfonyl(C₁₋₈) alkyl, amino, (C₁₋₈)alkylamino, di(C₁₋₈)alkylamino with two identical or different (C₁₋₈)alkyl moieties, amino(C₁₋₈)alkyl, (C₁₋₈)alkylamino(C₁₋₈)alkyl, di(C₁₋₈)alkylamino(C₁₋₈)alkyl with two identical or different (C₁₋₈)alkyl moieties in the di(C₁₋₈)alkylamino moiety, amino(C₁₋₈)alkoxy, (C₁₋₈) alkylamino(C₁₋₈)alkoxy, di(C₁₋₈)alkylamino(C₁₋₈)alkoxy with two identical or different (C₁₋₈)alkyl moieties, formyl, (C₁₋₈)alkylcarbonyl, formyloxy, (C₁₋₈) alkylcarbonyloxy, formyl(C₁₋₈)alkyl, (C₁₋₈)alkylcarbonyl(C₁₋₈)alkyl, formyl(C₁₋₈)alkoxy, (C₁₋₈)alkylcarbonyl(C₁₋₈)alkoxy, (C₁₋₈)alkoxycarbonyl, (C₁₋₈)alkoxycarbonyloxy, (C₁₋₈)alkoxycarbonyl(C₁₋₈)alkyl, (C₁₋₈) alkoxycarbonyl(C₁₋₈)alkoxy, -OCH₂O-, -C(=O)OCH₂-, -CH₂OC(=O)- and - CH=CHCH=CH-, the four last-mentioned optional substituents in each case being attached to two adjacent ring carbon atoms of the said moiety and
wherein said (C₁₋₈)alkyl group is unsubstituted or mono-, di-, tri or tetra-substituted, the optional substituent(s) on the said (C₁₋₈)alkyl moiety being independently selected from the group consisting of halogen, cyano, oxo, (C₁₋₈)alkoxy, (C₁₋₈)alkoxy(C₁₋₈)alkoxy, (C₁₋₈)alkylthio, (C₁₋₈)alkylsulfinyl, (C₁₋₈) alkylsulfonyl, (C₁₋₈)alkylcarbonyloxy, (C₁₋₈)alkoxycarbonyl and (C₁₋₈) alkoxy carbonyloxy.

3. The compound of formula (I), wherein
R¹ represents hydrogen, a (C₁-C₈)alkyl group, an aryl group, wherein said aryl group is unsubstituted,
wherein said alkyl group is unsubstituted, or mono-substituted, the optional substituent being selected from (C₂₋₄)alkenyl, (C₂₋₄)alkynyl, (C₁₋₈)alkoxy, phenyl, naphthyl; and
R² represents an aryl group or a (C₃-C₈)cycloalkyl group or a heteroaryl group with 5 or 6 ring atoms, or a heterocyclyl group with 5 or 6 ring atoms or a (C₁-C₈)alkyl group
which is unsubstituted or mono-, di-, tri- or tetra-substituted on the aryl group, the optional substituent(s) on said moiety being independently selected from the group, consisting of halogen, cyano, (C₁₋₈)alkyl, (C₁₋₈) alkyl substituted by halogen, nitro, (C₁₋₈)alkoxy, (C₁₋₈)alkoxy substituted by halogen, (C₁₋₈)alkylthio, formyloxy, (C₁₋₈)alkylcarbonyloxy;
which is unsubstituted or mono-, di-, tri- or tetra-substituted on the (C₃-C₈)cycloalkyl group, the optional substituent(s) on said group being independently selected from the group, consisting of halogen, cyano, (C₁₋₈) alkyl, (C₁₋₈)alkyl substituted by halogen, nitro, (C₁₋₈)alkoxy, (C₁₋₈)alkoxy substituted by halogen, (C₁₋₈)alkylthio, formyloxy, (C₁₋₈)alkylcarbonyloxy; which is unsubstituted or mono-, di-, tri- or tetra-substituted on the heteroaryl group, the optional substituent(s) on the said group being independently selected from the group, consisting of halogen, cyano, (C₁₋₈) alkyl, (C₁₋₈)alkyl substituted by halogen, nitro, (C₁₋₈)alkoxy, (C₁₋₈)alkoxy substituted by halogen, (C₁₋₈)alkylthio, formyloxy, (C₁₋₈)alkylcarbonyloxy; and whereby the heteroarylmoiety is contains 1-3 nitrogen atoms or 0-2 nitrogen and one oxygen or sulfur atom;
which is unsubstituted or mono-, di-, tri- or tetra-substituted on the heterocyclyl group, the optional substituent(s) on the said group being independently selected from the group, consisting of halogen, cyano, (C₁₋₈) alkyl, (C₁₋₈)alkyl substituted by halogen, nitro, (C₁₋₈)alkoxy, (C₁₋₈)alkoxy substituted by halogen, (C₁₋₈)alkylthio, formyloxy, (C₁₋₈)alkylcarbonyloxy; and whereby the heterocyclylmoiety is contains 1-3 nitrogen atoms or 0-2 nitrogen and one oxygen or sulfur atom which is unsubstituted in the (C₁-C₈)alkyl group.

4. The compound of formula (I), wherein
R¹ represents hydrogen; and
R² represents phenyl or naphthyl, which is unsubstituted or substituted by one or two substituents on the phenyl or naphthyl group, the optional substituents on said group being independently selected from fluoro, chloro, bromo, methyl, ethyl, propyl, tert-butyl, trifluormethyl, methoxy, ethoxy and trifluormethoxy.

5. A compound of formula (II) 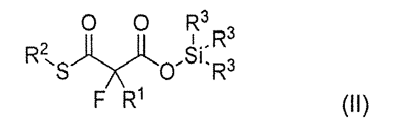 wherein
R¹ is as defined in any of claims 1 - 4;
R² is as defined in any of claims 1 - 4;
R³ represents, independent from each other, a (C₁-C₈)alkyl group, a (C₁-C₈)alkoxy group, an aryl group, an aryl substituted by (C₁-C₈)alkyl.

6. The compound of formula (II), wherein
R¹ is hydrogen;
R² phenyl or naphthyl, which is unsubstituted or substituted by one or two substituents on the phenyl or naphthyl group, the optional substituents on said group being independently selected from fluoro, chloro, bromo, methyl, ethyl, propyl, butyl, trifluormethyl, methoxy, ethoxy and trifluormethoxy,
R³ represents independent from each other methyl, ethyl, propyl, butyl, phenyl.

7. A method for manufacturing a compound of formula (I) according to any of claims 1 - 4, said method comprising the steps of
(a) reacting a compound of formula (III) with a compound of formula (IV) 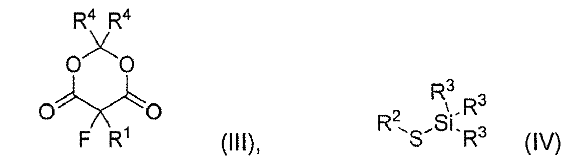 wherein
R¹ is as defined in any of claims 1-4,
R² is as defined in any of claims 1-4,
R³ is as defined in any of claims 5 or 6
R⁴ independently represents a (C₁-C₈)alkyl group, hydrogen, (C₁-C₈)cycloalkyl group, or both R⁴ together with the carbon atom attached to represent (C₃-C₈)cycloalkyl group;
optionally in the presence of a diluent and/or a reaction aid;
(b) hydrolysing the thus obtained compounds.

8. The method of claim 1, wherein step (a) takes place in tetrachlorethane as a diluent and / or @ temperatures between 70 - 100°C.

9. Use of a compound according to any of claims 1-4 as a nucleophile.

10. Use of a compound according to any of claims 1-4 in in nucleophilic addition reaction or in a nucleophilic substitution reaction.

11. The use according to claim 10, wherein said reaction being selected from the group consisting of
(a) addition to aldehydes;
(b) addition to imines;
(c) conjugate additions to nitroolefines;
(d) conjugate additions to α,β unsaturated aldehydes, ketones, or esters;
(e) nucleophilic substitution of alkyl halides or sulfonates.

12. The use according to any of claims 10 or 11, which is an enantioselective reaction.

13. The use according to any of claims 10 to 12, wherein a diluent and / or an reaction aid, particularly a chiral catalyst, is present.
